# EUROPEAN PATENT APPLICATION

(11) **EP 1 524 626 A2**
(43) Date of publication of application: **20.04.2005**
(21) Application number: 04254228.2
(22) Date of filing: 14.07.2004
(51) Int. Cl.: G06T 7/00

(54) **Optical image-based position tracking for magnetic resonance imaging**

(30) Priority: 14.07.2003 US 487402
(71) Applicant: Sunnybrook & Women's College Health Sciences Centre, Toronto, Ontario M4N 3M5 (CA)
(72) Inventor: Tremblay, Marleine, Toronto Ontario M6B 1B1 (CA); Tam, Fred, Toronto Ontario M4K 2A2 (CA); Graham, Simon James, Toronto Ontario M4G 2J5 (CA); Kucharczyk, John, Bishop, GA 30621 (US)
(74) Representative: Bowman, Paul Alan

(57) **Abstract**

An optical image-based tracking system determines the position and orientation of objects such as biological materials or medical devices within or on the surface of a human body undergoing Magnetic Resonance Imaging (MRI). Three-dimensional coordinates of the object to be tracked are obtained initially using a plurality of MR-compatible cameras. A calibration procedure converts the motion information obtained with the optical tracking system coordinates into coordinates of an MR system. A motion information file is acquired for each MRI scan, and each file is then converted into coordinates of the MRI system using a registration transformation. Each converted motion information file can be used to realign, correct, or otherwise augment its corresponding single MR image or a time series of such MR images. In a preferred embodiment, the invention provides real-time computer control to track the position of an interventional treatment system, including surgical tools and tissue manipulators, devices for in vivo delivery of drugs, angioplasty devices, biopsy and sampling devices, devices for delivery of RF, thermal energy, microwaves, laser energy or ionizing radiation, and internal illumination and imaging devices, such as catheters, endoscopes, laparoscopes, and like instruments. In other embodiments, the invention is also useful for conventional clinical MRI events, functional MRI studies, and registration of image data acquired using multiple modalities.

## Description

### RELATED U.S. PATENT APPLICATION DATA

This application claims priority from Provisional U.S. Patent Application Serial No. 60/487,402, filed 14 July 2003.

### BACKGROUND OF THE INVENTION

### 1: Field of the Invention

The present invention relates to magnetic resonance imaging (MRI), and more particularly to the use of an MRI-compatible optical position tracking method and apparatus.

### 2. Background of the Invention

Advances in medical imaging technology, including computerized tomography (CT), magnetic resonance imaging (MRI), and positron emission tomography (PET), coupled with developments in computer-based image processing and modeling capabilities have led to significant improvements in the ability to visualize anatomical structures in human patients, and to use this information in diagnosis, treatment planning and, most recently, real-time interventional procedures. The introduction of MRI into clinical practice in the early 1980's has had significant impact on the diagnosis and treatment of various diseases. Superb image contrast for soft tissues and millimeter scale spatial resolution have established MRI as a core imaging technology in most medical centers. MRI is unique among imaging modalities in that any one of a multitude of tissue properties can be extracted and highlighted. Anatomy can be defined in great detail, and several other biophysical and metabolic properties of tissue, including blood flow, blood volume, elasticity, oxygenation, permeability, molecular self-diffusion, anisotropy, and water exchange through cell membranes, can also be represented in MR images. Although conventional anatomical MR imaging using spin-echo, gradient-echo, and inversion recovery sequences continues to be the mainstay of clinical practice, there is a rapidly escalating array of other MR methods, including: magnetic resonance spectroscopy (MRS), apparent diffusion coefficient (ADC) mapping, diffusion-weighted imaging (DWI) and its derivatives of diffusion tensor imaging and tractography, perfusion imaging, permeability imaging, MR angiography (MRA), and functional MRI (fMRI).

As the clinical applications of MRI expand, there is a concurrent requirement for improved technology to visualize and determine the position and orientation of moving objects in the imaging field, including, for example, both biological materials and medical devices. Improvements in position tracking technology are required to advance four broad areas of MRI: 1) imaging of anatomy (e.g., tissue morphology and lesion characterization); 2) imaging of tissue function (e.g., physiologically-based parameters such as perfusion, or metabolite concentration); 3) interventional applications (e.g., image-guided minimally invasive therapies such as surgical resection, thermal-therapy, cryotherapy, and drug delivery); and 4) registration of MRI data with that of other imaging modalities, for detecting and diagnosing diseases, and for subsequent MRI-guided treatment planning and monitoring. In the context of the present invention, "coregistration" is defined as the alignment of images acquired with the same modality to a common spatial reference, whereas "registration" is defined as the analogous alignment procedure performed across different imaging modalities.

The rationale for the present invention is subsequently discussed with respect to the four application areas of MRI described immediately above. With anatomic MR imaging, the presence of moving biological tissue can be highly problematic because it can produce image artifacts, obscure the detection of lesions, and more generally complicate the interpretation of MR images. The time scale for acquiring diagnostic MRI typically ranges from several seconds to several minutes, which can yield significant postural, cardiac, respiratory, and blood flow image artifacts that can confound the ability to detect pathology. The typical appearance of such artifacts takes the form of 'blurring,' or a characteristic "motion ghost" in the phase encoding direction associated with incorrectly encoding the spatial frequencies of a moving object that is assumed to be static.

MR images of different body parts are contaminated differently by motion. Neuroimaging generally is less severely affected by motion artifacts than abdominal imaging, and cardiac imaging is most affected. For example, motion artifacts due to normal or abnormal respiratory movements can degrade image quality in MR scans where the patient is either allowed to breathe freely, breathes inadvertently, or if the MR study requires scan times in excess of a patient's ability to hold their breath. In these cases, some technique other than simple breath-holding must be used to minimize respiratory motion artifacts. Prior art methods of detecting such positional changes have relied upon navigator-type sequences which use MR to image periodically a two-dimensional column of spins that include the diaphragm. By detecting changes in the diaphragm position, data acquisition can be synchronized to a common position in the respiratory cycle. In this manner, MR data acquisition is gated to a specific position of the diaphragm, and by implication, to a specific position of the internal organs in the thoracic and abdominal cavities.

U.S. Patent No. 6,067,465 to Foo et al. discloses a method for detecting and tracking the position of a reference structure in the body using a linear phase shift to minimize motion artifacts in magnetic resonance imaging. In one application, the system and method are used to determine the relative position of the diaphragm in the body in order to synchronize data acquisition to the same relative position with respect to the abdominal and thoracic organs to minimize respiratory motion artifacts. The system and method use the time domain linear phase shift of the reference structure data to determine its spatial positional displacement as a function of the respiratory cycle. The signal from a two-dimensional rectangular or cylindrical column is first Fourier-transformed to the image domain, apodized or bandwidth-limited, converted to real, positive values by taking the magnitude of the profile, and then transformed back to the image domain. The relative displacement of a target edge in the image domain is determined from an auto-correlation of the resulting time domain information.

Another prior art method uses the phase of the echo peak, or the center of the k-space phase, as an indication of the relative displacement of the reference object. Although this has been used to correct for motion-related artifacts in functional neuroimaging studies, such a method cannot monitor diaphragmatic motion where a projection profile includes moving structures (liver, stomach, etc.) and slightly moving structures (lung, shoulder). This method also requires manual input of an initial positional selection by an MRI operator. Therefore, it would be desirable to have a system and method for detecting and tracking positional changes in a reference structure that is computationally efficient, is not reliant on operator input or influence, or on pixel size, and eliminates the need to require a patient to breath-hold, thereby eliminating an additional patient stress factor during a MRI procedure.

In the case of MR neuroimaging, the inability of the subject simply to remain still during the examination period may significantly compromise MR scan quality. High-spatial resolution is a basic requirement of 3D brain imaging data for patients with neurological disease, such as Parkinson's disease, stroke, dementia, or multiple sclerosis, and consequently motion artifacts may pose a significant problem Furthermore, there is often a need in such applications to look for changes in brain images over long periods of time (days, weeks, months), such as the waxing and waning of MS lesions, progressive atrophy in a patient with Alzheimer's disease, or the growth or remission of a brain tumor. In these cases, the ability to determine the position of anatomy as a function of scanning session is extremely important to enable coregistration and to detect and quantify subtle changes. Ideally, to image with the same spatial resolution and orientation in different examinations, it would be best to develop technology that enabled MRI scans of such subjects to be performed with the anatomy in precisely the same location within the MRI scanner on each session.

The ability to track motion in a "time series" of images is essential for a number of different MRI applications. For example, motion artifact suppression techniques have been useful in coronary artery imaging such as MRA, in fMRI, and in diffusion imaging. Another application is the monitoring of heart wall motion which is useful to assess the severity and extent of damage in ischemic heart disease. MR angiography of the coronary arteries has typically been performed using a technique to limit the MRI acquisition to avoid motion artifacts. Such techniques include requiring the patient to withhold breathing during the imaging, using oblique single-sliced image techniques, or respiratory-gated 3D imaging techniques. However, repeated breath holding may not be feasible for many coronary patients and navigation techniques to-date have not generally provided a robust method which works over a range of different breathing patterns in a variety of patients. Another drawback to these approaches is that success or failure is usually not apparent for some time after the start of imaging, and many times not until the imaging has been completed.

Another application requiring accurate compensation for anatomic movement includes myocardial perfusion imaging to detect the passage of a contrast agent through muscle tissue in the heart and to study the blood flow in the micro-circulation of the heart non-invasively. Typically, perfusion imaging consists of using injected contrast agents together with rapid imaging during the first pass of the contrast agent through the microvasculature with carefully optimized pulse-sequence parameters. Quantification of blood flow from these images is carried out with a region of interest-based signal, time-intensity curve analysis. To avoid cardiac motion artifacts, the perfusion images are typically acquired with ECG gating. However, since the period of image acquisition is usually 1-2 minutes long, the images suffer from significant respiratory motion artifacts. This then requires a manual registration and analysis of the perfusion images, which is cumbersome and time-consuming because the user must carefully arrange each image to compensate for the respiratory motion before proceeding to a region of interest time-intensity analysis.

Many of the advantages of MRI that make it a powerful clinical imaging tool are also valuable during interventional procedures. The lack of ionizing radiation and the oblique and multi-planar imaging capabilities are particularly useful during invasive procedures. The absence of beam-hardening artifacts from bone allows complex approaches to anatomic regions that may be difficult or impossible with other imaging techniques such as conventional CT. Perhaps the greatest advantage of MRI is the superior soft-tissue signal contrast available, which allows early and sensitive detection of tissue changes during interventional procedures.

In the case of interventional MRI, there is a requirement to place instruments accurately within the field of view (FOV) or near the FOV of image acquisition. Examples in the MRI environment include the location of interstitial probes to provide high-temperature thermal therapy, cryotherapy, or drug therapy for tumors while sparing surrounding normal tissues; location of non-invasive focused ultrasound probes for thermal therapy below the tissue surface; and the subcutaneous or transdural placement of biopsy needles or surgical instruments for minimally-invasive surgery. Exemplary of such endoscopic treatment devices are devices for endoscopic surgery, such as for laser surgery disclosed in U.S. Patent No 5,496,305 to Kittrell et al, and biopsy devices and drug delivery systems, such as disclosed in U.S. Pat 4,900,303 and U.S. Patent No. 4,578,061 to Lemelson.

MRI-guided interventional placements typically require a physician to be present but can also be actuated by assistive devices (e.g., robots). A key requirement in minimally-invasive or noninvasive procedures is to integrate the positioning of these instruments, needles, or probes with image guidance to confirm that the trajectory or location is as safe as possible, and to provide images that enhance the ability of the physician to distinguish between tissue types. Placement may require acquisition of static images for planning purposes, either in a prior MRI examination or during the interventional MRI session, or real-time images in arbitrary scan planes during the positioning process. ( Daniel et al. SMRM Abstr. 1997; 1928; Bomert et al. SMRM Abstr. 1997; 1925; Dumoulin et al. Mag Reson Med 1993; 29: 411-415; Coutts et al., Magnetic Resonance in Medicine 1998, 40:908-13)

Minimally-invasive interventional procedures require either direct visual viewing or indirect imaging of the field of operation and determination of the location and orientation of the operational device. For example, laparoscopic interventions are controlled by direct viewing of the operational field with rigid endoscopes, while flexible endoscopes are commonly used for diagnostic and interventional procedures within the gastrointestinal tract. Vascular catheters are manipulated and maneuvered by the operator, with real-time X-ray imaging to present the catheter location and orientation. Ultrasound imaging and new real-time MRI and CT scanners are used to guide diagnostic procedures (e.g., aspiration and biopsy) and therapeutic interventions (e.g., ablation, local drug delivery) with deep targets. While the previous examples provide either direct (optical) or indirect (imaging) view of the operation field and the device, another approach is based on remote sensing of the device with mechanical, optical or electromagnetic means to determine the location and orientation of the device inside the body.

Computer-assisted stereotaxis is a valuable technique for performing diagnostic and interventional procedures, most typically neurosurgery, whereby real-time measurements of the device location are obtained in the same coordinate system as an image of the field of operation. The current location of the device and its future path are presented in real-time on the image and provide the operator with feed-back to manipulate the device with minimal damage to the organs. During conventional stereotaxis, the patient wears a special halo-like headframe, which provides the common coordinate system, and CT or MRI scans are performed to create a 3D computer image that provides the exact location of the target (e.g., tumor) in relation to the headframe. The device is. mechanically attached to the frame and sensors provide its location in relation to the head frame. When this technique is used for biopsy or minimally-invasive surgery of the brain, it guides the surgeon in determining where to make a small hole in the skull to reach the target. Newer technology is the frameless technique, using a navigational wand without the headframe. In this technique, a remote sensing system (e.g., light sources and sensors) provides the real-time location of the device with respect to the image coordinate system. However, both the stereotactic and the frameless techniques are typically limited to the use of rigid devices like needles or biopsy forceps, since their adequate operation requires either mechanical attachments or line-of-sight between the light sources and the sensors.

U. S. Patent Nos. 6,317,616 to Glossop and 6,725,080 to Melkent et al. are exemplary of the method and usage of optical position tracking technology using light reflected or emitted from tools of precise geometries affixed to anatomy or to medical instruments, for the general purpose of image-guided therapy. However, these patents do not consider use of such technology directly within the MRI environment, which poses significant engineering constraints: high ambient, static magnetic field; the need to maintain spatial magnetic field uniformity to well within parts per million over the pertinent anatomy of the patient; stringent suppression of spurious electromagnetic interference at the radiofrequency (RF) resonance of the MRI system; and confined space, typically within the narrow bore of a superconducting magnet.

Exemplary of remote sensing techniques based on electromagnetism is the method and apparatus disclosed by U.S. Patent No. 5,558,091 to Acker et al. to determine the position and orientation of a device inside the body. This method uses magnetic fields generated by Helmholtz coils, and a set of orthogonal sensors to measure components of these fields and to determine the position and orientation from these measurements. The measurement of the magnetic field components is based on the Hall effect and requires exciting currents in the sensors to generate the measured signals. The technique requires control of the external magnetic fields and either steady-state or oscillating fields, for the induced voltages to reach a state of equilibrium. These requirements prevent, or greatly complicate, the use of this technique with magnetic fields generated by the MRI system. Furthermore a dedicated set of coils is required to generate the necessary magnetic fields.

A different approach for remote sensing. of location is disclosed by U.S. Patent No. 5,042,486 to Pfeiler et al. and by U.S. Patent No. 5,391,199 to Ben Haim. This technology is based on generating weak RF signals from three different transmitters, receiving the signals through an RF antenna inside the device, and calculating the distances from the transmitters, which define the spatial location of the device. However, the application of this technology to MRI is problematic due to the simultaneous use of RF signals by the MR scanning. Potential difficulties are the heating of the receiving antenna in the device by the high amplitude excitation RF transmissions of the MRI scanner and artifacts in the MR image.

U.S. Patent No. 5,271,400 and No. 5,211,165 to Dumoulin et al. disclose a tracking system employing magnetic resonance signals to monitor the position and orientation of a device within a human body. The device disclosed by Dumoulin's invention has an MR-active sample and a receiver coil which is sensitive to MR signals generated by the MR-active sample. These signals are detected in the presence of MR field gradients and thus have frequencies which are substantially proportional to the location of the coil along the direction of the applied gradient. Signals are detected by sequentially applied, mutually orthogonal magnetic gradients to determine the device's position in several dimensions. The position of the device as determined by the tracking system is superimposed upon independently acquired medical diagnostic images. However, this method may be subject to heating of the coil, and requires time to implement that reduces the temporal resolution available for repeated MRI acquisitions.

The patented inventions referenced above provide useful aids for introducing and delivering interventional devices to specific targets in the body. However, each invention also has significant inherent limitations. The ideal system for minimally invasive procedures should provide real-time, 3D imaging as feedback to the user for optimal insertion and intervention. Such a system should also implement flexible, miniaturized devices which are remotely sensed to provide their location and orientation. By combining a composite image of the field of operation and the device location and orientation, the operator could navigate and manipulate the device without direct vision of the field of operation and the device.

The use of MRI to measure physiologic and metabolic properties of tissue non-invasively requires dynamic imaging to obtain time-series data For example, functional magnetic resonance imaging (fMRI) to measure brain activity relies on a well-established neurovascular coupling phenomenon that results in transient increases in blood flow, oxygenation, and volume in the vicinity of neurons that are functionally activated above their baseline level. Signal changes due to the blood oxygenation-level-dependent (BOLD) effect are intrinsically weak (only several percent signal change from baseline at 4.0 T or less). In addition, as BOLD imaging is typically coupled with a repetitive behavioral task (e.g., passive sensory, cognitive, or sensorimotor task) to localize BOLD signals in the vicinity of neurons of interest, there is significant potential for fMRI to be confounded by the presence of small head motions. Specifically, such motion can introduce a signal intensity fluctuation in time due to intra-voxel movement of an interface between two different tissues with different MR signal intensities, or an interface between tissue and air. Random head motion decreases the statistical power with which brain activity can be inferred, whereas task-correlated motion cannot be easily separated from the fMRI signal due to neuronal activity, resulting in spurious and inaccurate images of brain activation. In addition, head motion can cause mis-registration between neuroanatomical MR and fMR images that are acquired in the same examination session. This latter point is important because the neuroanatomical MRI data serve as an underlay for fMRI color maps, and mis-registration results in mis-location of brain activity. An analogous problem exists for aligning anatomical and functional MR images performed on different days.

There is considerable published medical literature describing various aspects of motion detection and quantitation in fMRI, given the difficulty of the problem(e.g., Seto et al., *Neurolmage* 2001,**14**:284-297; Hajnal et al *Magn Res Med* 1994, **31**: 283-291; Friston et al., *Magn Res Med* 1996, **35**:346-355; Bullmore et al., *Human Brain Mapping* 1999, **7**: 38-48; Bandettini et al., *Magn Res Med* 1993,**30**:161-173; Cox. *Comp Med Res* 1996, **29**:162-173 ; Cox et al., *Magn Res Med* 1999, **42**:1014-1018 ; Grootoonk et al., *Neurolmage* 2000, **11**:49-57; Freire et al., *IEEE Trans Med Im* 2002, **21**(5):470-484; Babak et al., *Magn Res Im* 2001, **19**:959-963; Voklye et al. 1999, *Magn Res Med* **41**:964-972). Conversely, in some fMRI examinations anatomic motion is not a detriment, but instead is absolutely essential. In particular, fMRI of aspects of human motor system performance typically requires the patient to execute a movement as part of the behavioral task that is imaged to visualize brain activity. Medical applications for such imaging include fMRI of patients with brain tumors for the purpose of neurosurgical planning, and fMRI of patients recovering from stroke, to determine the most appropriate therapeutic strategy to promote recovery (selection of targeted physical therapy and/or drug therapy) on the basis of brain activity patterns. Movements can be very simple (e.g., self-paced finger tapping) or more complex (e.g., visually-guided reaching). Such examinations require both that the desired movement is performed in a well-controlled or well-quantified fashion, and also that the movement does not induce task-correlated head motion that confounds the ability to observe brain activity using fMRI. Perhaps the most complicated scenario involves combining use of virtual reality (VR) technology with fMRI, to determine brain activity associated with VR tasks for assessment and rehabilitation of impaired brain function. Such applications are important from the standpoint of "ecological validity" as they provide the opportunity to visualize brain activity associated with tasks that generalize well to everyday behavior in the real 3D-world. For example, position tracking would be required to provide realistic visual representation of a virtual hand operated by a data glove in a virtual environment.

For anatomical and functional MRI applications, as well as interventional MRI, there is the additional need to register data from other imaging modalities to provide comprehensive and complementary anatomical and functional information about the tissue of interest. The registration is performed either to enable different images to be overlaid, or to ensure that images acquired in different spatial formats (e.g., MRI, conventional x-ray imaging, ultrasonic imaging) can be used to visualize anatomy or pathology in precisely the same spatial location While some algorithms exist for performing such registrations, computational cost would be significantly reduced by developing technology that enables data from multiple different imaging modalities to be inherently registered by measuring the patient's orientation in each image with respect to a common coordinate system.

There are additional teachings in the literature regarding measurement techniques and images correction schemes. It is well known that motion between images acquired with MRI greatly reduces their utility and effectiveness. Motion correction techniques have been under continuous development since the initial development of MRI. Incremental improvements in motion artifact reduction have been achieved as the mechanisms behind motion artifacts in anatomical MRI have been increasingly understood. To date, however, no generally acceptable solution has been reported. Several approaches described in the medical and patent literature disclose methods to prevent motion corruption by manipulating MRI pulse sequences based on simple assumptions regarding the nature of the motion (temporal and frequency characteristics) to make MRI less motion-sensitive. The simplest approach is to average imaging data repetitively, although this reduces spatial resolution. To reduce the effect of respiratory motion, potential solutions described in the art include combining breath-holding and fast scan approaches; gating approaches to acquire MRI data only during a certain phase of the respiratory cycle; data acquisition re-ordering schemes to make the resulting images less sensitive to motion, and development of "spiral" and "gradient moment-nulled" imaging pulse sequences that are intrinsically motion-compensated due to the temporal pattern of gradient waveforms that is adopted for spatial encoding. With the exception of breath-holding, these techniques also apply to cardiac imaging, with the addition that real-time imaging is being developed particularly for this application to "freeze" cardiac anatomy within an image frame and to view the resultant data as a movie loop to evaluate cardiac status dynamically.

Prior art attempts at tracking motion using cross-correlation and other simple distance measurement techniques have not been highly elective where signal intensities vary either within images, between images, or both. In the context of the present invention, the term "signal intensity variations" should be understood to include variations over space and time, and to also include pixel by pixel changes both within an image and changes between images. Such signal variations arise regularly in MR imaging due to flow effects, motion effects, wash-through of contrast agents, and movement of anatomy through an image slice, among other reasons. The present invention solves the aforementioned problems with a local pattern matching technique that is insensitive to signal intensity variations in and between MR images. Rather, the pattern matching involves imaging markers in a rigid geometrical arrangement that are placed on the object to be tracked.

U. S. Patent No 6,292,683 to Gupta et al. discloses a method and apparatus to track motion of anatomy or medical instruments between MR images. The invention includes acquiring a time series of MR images of a region of interest, where the region of interest contains the anatomy or structure that is prone to movement, and the MR images contain signal intensity variations. The invention includes identifying a local reference region in the region of interest of a reference image and acquired from the time series. The local reference region of the reference image is compared to that of the other MR images and a translational displacement is determined between the local reference region of the reference image and of another MR image. The translational displacement has signal intensity invariance and can accurately track anatomy motion or the movement of a medical instrument during an invasive procedure. The translational displacement can be used to align the images for automatic registration, such as in myocardial perfusion imaging, MRA, fMRI, or in any other procedure in which motion tracking is advantageous. Two implementations of the invention are disclosed, one in which a correlation coefficient is calculated and used to determine the translational displacement, and one in which the images are converted to a binary image by thresholding (using signal intensity thresholds) and after computation of a filtered cross-correlation, a signal peak is located and plotted as the translational displacement. However, unlike the present invention, the method disclosed by Gupta is entirely image-based, relies on the identification of an appropriate reference region of interest (if one in fact exists) and provides position tracking at a maximum rate dictated by the temporal resolution of the image time series, such that within-image motion corrections are not possible. Examples of these techniques are shown in U.S. Patent Nos. 5,947,900 (Derbyshire) and 6,559,641 (Thesen)

U.S. Patent No. 6,516,213 to Nevo discloses a method and apparatus to determine the location and orientation of an object, for example a medical device, located inside or outside a body, while the body is being scanned by magnetic resonance imaging (MRI). More specifically, the invention by Nevo enables estimation of the location and orientation of various devices (e.g., catheters, surgery instruments, biopsy needles) by measuring voltages induced by time-variable magnetic fields in a set of miniature coils, said time-variable magnetic fields being generated by the gradient coils of an MRI scanner during its normal imaging operation. However, unlike the present invention, the system disclosed by Nevo is not capable of position tracking when imaging gradients are inactive, nor is it capable of measurements outside the sensitive volume of the imaging gradients (i.e., significantly outside the magnet bore in the static fringe magnetic field of the MRI system, or even outside the magnet room entirely).

Other strategies require the identification and accurate measurement of motion as a prerequisite for subsequent suppression of motion-induced artifacts. The technique of "navigator echoes" was originally developed to measure the one-dimensional movement of internal abdominal organs (e.g., liver) as a basis for correcting MRI "raw data in k-space", prior to Fourier-transformation to obtain anatomical images. In the case of neuroanatomical MRI and in comparison to other anatomical imaging of the abdomen or the heart, movement of the head most closely resembles simple rigid-body motion. This permits use of various coregistration algorithms that assume rigid-body rotations and translations to MR images to estimate the underlying head motion based on minimization of a performance metric, or "cost function". Similar algorithms have been developed for the registration of tomographic images acquired by different modalities. An output of all such algorithms is an estimate of the head motion between the different images of a time series. However, an independent, direct measurement of head motion could also be used for coregistration purposes, rather than using estimates.

A subset of all of the above correction schemes is currently conventionally employed in fMRI. As in anatomical MRI, these schemes remain an incomplete solution to the problem and the search for improved motion suppression continues. Typically, fast imaging is employed to "freeze" motion within the fMRI acquisition time frame (typically temporal resolution of several seconds), in combination with use of head restraints to limit motion. Subsequently, image-based, retrospective coregistration is used to realign fMR images as a function of time. In practice, this approach works quite well in compliant patients. However, it is still possible to achieve poor activation image quality if patients exhibit task-correlated motion on the order of 1 millimeter. This problem is particularly manifest in specific patient populations (e.g. dementia, immediate post-acute phase of stroke). Furthermore, image-based coregistration algorithms suffer from methodological limitations. They typically perform at the temporal resolution of the image time series to be co-registered (no intra-image motion is possible); they are sensitive to confounding signal fluctuations (e.g., eye movement, motion of the brain stem with cardiac and respiratory cycles) that can be misconstrued as rigid body motion, and they are sensitive to image quality parameters such as spatial resolution, signal contrast, and signal-to-noise ratio. Consequently, the resulting co-registered images still can suffer from residual motion contamination that impairs the ability to interpret brain activity.

Recently, "real-time" fMRI approaches have been advocated that provide images of brain activity during fMRI data acquisition. primarily to Judge that the fMRI data are of sufficient quality and uncontaminated by motion The judgment is typically made based on the appearance of activation images, or from visual display of motion estimates obtained by coregistration algorithms. In the event of excessive motion, the scanning potentially can be repeated. Other real-time applications are being developed, including prospective coregistration algorithms to ensure that the scan plane remains in a fixed orientation and position with respect to the moving head. This approach has been shown to be effective and requires a measurement of head motion A variety of different implementations exist, using navigator echoes, laser tracking systems, and image-based coregistration algorithms to estimate head position and orientation.

In an alternative real-time approach, it is also possible to provide patients with visual feedback of their head position where they are instructed to remain still. This has been shown to reduce head motion and actively engages the patient in remaining vigilant. However, it increases attentional demands and consequently modulates fMRI signals of brain activity, and may therefore not be broadly applicable across patient populations.

There are also several drawbacks to the use of an external, MRI-compatible position-tracking device. Such measurements are inherently limited to sensing the motion at the surface of an object, not the interior. Motion of internal anatomy can only be inferred by its affect at the skin surface. Another limitation is the necessity to transform the position data into the co-ordinate system of MR image acquisition. Another aspect of the present invention is therefore to overcome partly such problems and limitations through the development of a calibration procedure and tracking the position of multiple tools.

### SUMMARY OF THE INVENTION

The present invention relates to an optical image-based tracking system that senses the position and orientation of objects such as, by way of non-limiting example, biological materials and medical devices within a surgical cavity or on the surface of a patient undergoing MRI. In the method of the invention, a reference tool is fixed to a stationary target as close as possible to the centre of the sensitive measuring volume of an MRI-compatible camera system. According to the invention, a second ''tracking'' tool, comprising an assembly of reflective markers having a different geometry than the reference tool, so as to allow the camera system to distinguish between the reference tool and the tracking tool, is rigidly mounted on a stationary phantom (test object). In the method of the invention, the tracking tool has its holes titled with an aqueous solution of MR contrast agent. By titling is meant that a specific indication/marking/signal is provided that specifically and uniquely identifies or distinguishes individual holes. In various alternative practices of a method of the invention, a plurality of precisely separated cameras, which are MRI-compatible with respect to ferromagnetic properties and electromagnetic interference at the Larmor frequency of the MRI system, are placed within line-of-sight of the tools (and thus the object) to be tracked. According to the invention, a high resolution MR image of the phantom with the tracking tool mounted on the Phantom is acquired, while the camera tracks its 3D configuration as well as that of the reference tool. The 3D positions of all holes are obtained both in the MR system's coordinates and the camera system's coordinates. From knowledge of the 3D coordinates of a set of points in two different coordinate systems, the registration transformation between the two coordinate systems is recovered using Horn's closed solution using quaternions (B.K.P. Horn, *J Opt Soc Am A* **4**: 629-642, 1987). After all necessary information is obtained for the registration of the two different systems, (for example, the MR systems and camera's coordinate systems) anatomical, functional, or interventional MRI examinations are subsequently undertaken with the tracking tool mounted to the object of interest. Acquisition of the position tracking data is triggered to the MR systems imaging acquisition. The position tracking data are then converted into coordinates of the MRI system using the registration transformation. The position tracking data can then be used to realign the corresponding functional MRI time series of images, to correct or augment MR anatomical images, to assist in interventional MRI, and to register MRI data with analogous imaging data acquired by alternate imaging modalities. Unlike other prior art, (i.e., motion tracking technology which performs inadequately when signal variations arise in MR imaging due to flow effects, motion effects, or wash-through of contrast agents) the present invention discloses a local pattern matching technique that is insensitive to signal variations in and between MR images through use of rigidly mounted reflective makers.

One aspect of this invention is to provide an MRI-compatible optical position tracking system to improve MRI data quality.

A second aspect of the present invention is to provide an MRI-compatible optical position tracking system for anatomical and functional MRI of biological tissues.

A third aspect of this invention is to provide an MRI-compatible optical position tracking system for interventional MRI applications where images are used to guide and monitor minimally-invasive diagnostic and therapeutic procedures.

A further aspect of this invention is to provide an MRI-compatible optical position tracking system for applications that require accurate registration of MRI data and with data obtained using other imaging modalities.

Yet another aspect of the present invention is to provide an MRI-compatible optical position tracking system to evaluate changes longitudinally in brain images acquired over long periods of time: days, weeks, and months.

Another aspect of this invention is to provide a system and method for detecting and tracking positional changes in a reference structure that is computationally efficient, is not reliant on operator input or influence, or on pixel size, and eliminates the need to require a patient to breath-hold, thereby eliminating an additional patient stress factor during an MRI procedure.

Still another aspect of the invention is to provide a motion tracking system with a local pattern matching technique that is insensitive to signal variations in and between MR images.

A further aspect of the present invention is to provide a position-tracking device whose function is independent of the MR scanner, such that position tracking data can be acquired at a rate permitted by the camera system.

It is another aspect of this invention is to provide a motion tracking system with the ability to determine the position of anatomy as a function of scanning session to enable coregistration and to detect and quantify subtle changes.

It is yet another aspect of the present invention to provide a motion tracking system which enables MRI scans to be performed with the anatomy in precisely the same location within the MRI scanner on each session to permit MR imaging with the same spatial resolution and orientation in different examinations.

Still another aspect of the present invention is to provide an optical position tracking method to co-register neuroanatomical MRI with fMRI images of brain activity.

A further aspect of this invention is to provide a method of position tracking to validate image-based coregistration algorithms.

Another aspect of this invention is to provide an optical position tracking system with real-time computer control to sense and maintain the position of an interventional treatment system for use with surgical tools and tissue manipulators, devices for in vivo delivery of drugs, angioplasty devices, biopsy and sampling devices, devices for delivery of RF, thermal, microwave or laser energy or ionizing radiation, and internal illumination and imaging devices, such as catheters, endoscopes, laparoscopes, and like instruments.

### Brief Description of the Drawings

FIG. **1A** illustrates the position and arrangement of multiple MRI-compatible cameras in relation to tracking and reference "tools".
FIG. **1B** illustrates the tracking tool, which contains optically-reflective markers as well as holes that can be filled with contrast agent material for visualisation using MRI.
FIG. **2** illustrates potential options for placement of the tool on a patient's head for tracking head movement. A) Hat configuration. B) Bite bar configuration. C) Molded face-mask configuration.
FIG. **3** is a block diagram describing the integration of the camera with MRI scanner hardware.
FIG. **4** shows A) representative spatial coordinates of reflective markers and holes for a specific tracking tool, and B) a high resolution MRI of the same tool for visualization of hole positions.
FIG. **5** shows the interior of the magnet bore of an MRI system, from the visual perspective of the tracking system camera (spatial coordinate frame shown in bottom left). The subject performs a bilateral finger tapping and visually-guided tracking experiment in which head motion measured from the tracking tool is used to move a black cursor laterally and in synchrony with an open circle. See Detailed Description of the Invention for further details. For clarity, the head coil and stationary reference tool are not shown.
FIG **6** shows representative plots of head motion in six degrees-of-freedom obtained by camera-based tracking (black) and by image-based coregistration (gray).
FIG **7** shows A) representative images of brain activity associated with the tapping and tracking task obtained with image-based coregistration using AFNI software (left column) and with coregistration by camera-based tracking (right column). Also shown are B) voxel histograms (number of activated voxels vs. fMRI BOLD signal intensity) for image-based coregistration (gray) and coregistration by camera-based tracking (black). Overall, the activation images and histograms are quite similar, although the tracking approach results in fewer activated voxels throughout the brain.
FIG. **8** shows histograms of the number of voxels significantly correlated with the predominant head motion (roll), for analysis without coregistration (dotted lines), with image-based coregistration (gray), and coregistration with camera-based tracking (black). Without coregistration, task-correlated motion is extensive, whereas both coregistration approaches provide approximately ten-fold suppression of task-correlated motion.
FIG. **9** shows A) the gradient echo k-space data (log 10 of magnitude) and B) corresponding MR image of a static gel phantom containing plastic bolts, nuts, and washers.
FIG. **10** shows A) rotation of the phantom in FIG. **9** as measured by the camera-based tracking system during gradient echo imaging acquisition. Motion is transformed to scanner coordinates and is predominantly in the roll direction. B) Estimated k-space traversed based on the motion data in A), assuming only roll rotation.
FIG. **11** shows A) the gradient echo k-space data measured for the phantom undergoing the rotation shown in FIG. **10.** Under the assumption of no motion, as commonly adopted in conventional MR imaging, the data are assumed to lie on a rectilinear grid in k-space. Significant distortion is present in comparison with FIG. **9A.** B) Corresponding MR image exhibiting motion artifact.
FIG. **12** shows A) the gradient echo k-space data corrected for motion according to the estimated trajectory shown in FIG. **10,** including gridding to Cartesian coordinates. B) Corresponding MR image showing substantial reduction of motion artifact. Gridding results in a slight loss in spatial resolution, in comparison with the MR image shown in FIG. **9.**

These and other features, objects, and advantages of this invention will be obvious upon consideration of the following detailed description of the invention. It will also be apparent to those of ordinary skill in the art that many changes and modifications may be made without departing from the scope of the present invention.

### DETAILED DESCRIPTION OF THE INVENTION

Clinical applications of this invention can be broadly divided into diagnostic MR imaging and interventional MRI. Artifacts due to patient movement are often a major problem in diagnostic MR imaging. With high-resolution scanning, which may require image acquisition over many seconds and even minutes, patient movement and breathing may induce motion artifacts and blurred images. MR scanning is specifically sensitive to movements during phase contrast angiography, diffusion imaging, and functional MRI with echo-planar imaging (EPI) or spiral imaging. According to the present invention, real-time determination of the location and orientation of the scanned object can reduce the effect of motion on MR scans by real-time control and correction of the scanning plane.

The invention may be contemplated as a method of relating movement of a patient (or a segment of a patient such as the interior or exterior of a head, thorax, thigh, neck, etc.) by independent measurement and detection of movement that can be directly or indirectly related to movement of the body segment that is of interest to the medical procedure performed under MRI visualization. By way of a non-limiting example, consider a visually observed point(s) or object(s) on the forehead of a patient. The object moves in a fixed relationship between the position of the object(s) on the forehead and the internal portion of the brain visualized by MRI techniques. As the object(s) are preferably external, they may be tracked in real time and measured according to actual time. The amount of movement from any base position (e.g., the original position of the head of the patient) can be determined at any time. The relationship of the segment of the body (here which portion of the brain is being considered for medical treatment) can be directly related to the position of the object(s). Even as the head moves in three dimensions, the relative position between the object(s) and the brain will not change. The MR scans are also identified with respect to the same time frame that is being used for the visual observation of the object(s). A simple geometric conversion of the image data of the MRI scan using the precisely known position of the object(s) will allow transformation of the data on the MR image affected by motion of the body segment to image data that is corrected for the determined and recorded gross or modest external movement. In this manner, the observed, recorded and detailed external movement provides a direct basis for correcting for motion effects on the MR image. The system may be alternatively described as a multi-modality imaging system comprising a MRI-compatible motion tracking system that is external to the patient, an MRI system and an MRI-compatible tool(s) that is both visible by MRI and that can be tracked in three dimensions, using the motion tracking system, in coordinates of the MRI system, the motion tracking system being referenced in time with respect to MRI data acquisition so that motion effects in the MR image(s) can be corrected. The tool comprises a device having holes that are titled with an MRI responsive material. The system preferably has movement of the tool tracked to provide information on the movement of an interventional medical device, or a body segment imaged using MRI. The motion of the body segment causes insignificant or inconsequential degrees of movement or any movement between the point(s) of reference/objects) and the tool.

### Advantages of MRI-compatible Position Tracking

There is a clear and growing need to provide enhanced technology for measuring motion in MRI and to reduce motion effects in images produced for medical purposes in MRI procedures. As MRI technology continues to advance rapidly, including the development of whole-body MRI at very high magnetic fields (approximately 3.0 T to 7.0 T), the resulting improvements in image signal contrast, spatial resolution, and signal-to-noise ratio will require increased ability to measure motion accurately for the purpose of motion artifact correction. Conversely, where motion is the central parameter of interest, continued improvements in measurement accuracy and flexibility will be extremely important.

Several benefits would result from the development of position-tracking devices that are independent from yet compatible with MR scanning. First, the MR exam would be completely independent from the measurements obtained by the device, such that position tracking data could be acquired at a rate permitted by the camera system, potentially exceeding that achievable on the MR system. Currently. the use of an MR system to perform position tracking typically requires additional time within an imaging pulse sequence for extraction of motion parameters, and this decreases the temporal resolution with which images can be acquired. Second, the development of a position-tracking device which is "transparent" with respect to MR procedures would allow standard methods for motion to be applied Jointly, either at the time of data acquisition or retrospectively . Third, the /one of optimal accuracy and sensitivity of the position tracking device could be made independent (and potentially larger) than the maximum field-of-view typical in MRI (approximately 45 cm). In addition to signal-to-noise ratio and presence of image artifacts, MR-based measurements of position are subject to non-idealities, such as nonlinearity of the gradient fields used to encode spatial position, and magnetic field non-uniformity due to the intrinsic shim of the superconducting magnet and patient-dependent magnetic susceptibility effects. As a result, position measurements that are optically-based could likely be acquired more accurately over a larger volume and could be used to track motions in the fringe magnetic field of the scanner, if required. Lastly, an independent position-tracking device could also be used to validate other approaches (either existing or future) for motion measurement and correction. Such technology would serve an immediate useful purpose, because most registration and coregistration algorithms have been validated on the basis of simulated data sets, or by comparison with other more established algorithms.

The present invention provides a number of specific advantages for optical imaging over other potential position tracking systems and devices. With two or potentially more well-calibrated cameras precisely separated (the dimensions in all relative parameters may be defined on the basis of physical differences in position, angular differences, focal plane differences, and temporal differences), it is possible on the basis of the camera geometry and parallax principles to estimate motion with six degrees of freedom of a rigid tool containing multiple precisely located reflective objects. Current charge-coupled-device (CCD) camera technology can easily be made MRI-compatible. This can yield images with high spatial resolution, and can operate readily at video frame rates. Over small measurement volumes typical of head motion, such a tracking technology can provide measurement accuracy and precision below 100 microns. Although this is not as accurate as would be possible using laser interferometry principles, laser-based systems require accurate line-of-site positioning of reflective mirrors that can be very time-consuming and difficult, and can pose a safety hazard to the eye associated with intense laser radiation. Using a camera-based system, a low-intensity pulsed or continuous light source is possible, sensitive to specific optical wavelengths (e.g., infrared) so that the human vision within the MRI system remains unaffected. No electrical cables are required within the magnet bore that could potentially be a source of electromagnetic interference with MR imaging acquisition.

The term "camera" as used herein is not limited in scope to any specific mechanism of operation for capture of the image from available radiation. Both analog and digital camera systems may be used, cameras sensitive to any available range of electromagnetic radiation may be used, and any capture mechanism (e.g., charge-coupled devices, small area arrays, large area arrays, semiconductor photoresponse systems, electrophotoconductor response, lens focused systems, direct light impact systems, mirror directed systems, and the like) known in the art may be used.

The method and apparatus of the present invention can also be used in interventional MRI with various devices, like miniature tools for minimally invasive surgery, endovascular catheters, rigid and flexible endoscopes, and biopsy and aspiration needles. The invention enables measurement of the location of the device with respect to the MRI coordinate system and allows the MR scanner to present the device location on the MR images as visual feedback to the operator, or to calculate and display the line of current orientation to assist the operator to steer the device into a specific target. The method of the invention can also be used to slave or subordinate the MRI plane of imaging to the tracking sensor. This embodiment would benefit high resolution imaging on a small volume around the site of a catheter, and would also be useful for imaging of the region-of interest to improve diagnostic performance or to control the effect of an intervention (e.g. RF energy, moderate energy treatments such as infrared or ultraviolet treatments, cryogenic, or chemical ablation and laser photocoagulation using temperature-sensitive MR imaging). Another embodiment, analogous to the use of optical endoscopes, is to employ information about the location and orientation of the reference object to display of the MRI images in relation to the local coordinate system, as if the operator were looking through the device and in the direction of the tip. A further clinical application of the invention is based on using the location tracking to mark locations of previous interventions on the MRI image.

The present invention may also have particular clinical utility in percutaneous myocardial revascularization (PMR) procedures. PMR is typically performed during cardiac catheterization. A laser transmitting catheter is inserted through the femoral artery, up through the aorta, and into the left ventricle of the heart. Based on prior perfusion studies (e.g., Thallium scan), or indirect information on viability of the myocardium (e.g., by measurement of local wall motion), the cardiologist applies laser energy to drill miniature channels in the inner portion of the heart muscle, which stimulates angiogenesis and new blood vessel growth. PMR potentially provides a less invasive solution (compared to bypass surgery) for ischemic heart disease patients which cannot be adequately managed by angioplasty or stent placement. It may also be used in conjunction with angioplasty or stenting to treat areas of the heart not completely re-vascularized by balloon- or stent-based interventions. Currently, PMR is exclusively done with X-ray guidance. The main advantage of MRI is the excellent performance of contrast-enhanced MRI in the assessment of myocardial blood perfusion. Thus, rather than relying on indirect information to localize poorly perfused myocardial tissues, a diagnostic MRI myocardial perfusion exam could be followed immediately by the appropriate therapeutic intervention using the existing MRI perfusion images and real-time tracking of the laser catheter and the tracking methodology disclosed by the present invention. An additional advantage of using MRI is the potential to control the intervention by high-resolution, real-time imaging of the myocardium during the application of the laser treatment. Since PMR is typically performed on multiple regions of the myocardium, marking the location of treated locations on the perfusion image based on the location of the catheter tip with respect to the location data of the tracking system of this invention provides a detailed map of the treated myocardium in relation to the overall anatomy of the heart. In addition and as mentioned previously, the tracking system also aids in this cardiac application through the ability to improve image quality by reduction of respiratory artifact.

The tracking system disclosed in the present invention can also be used for various diagnostic and interventional procedures within the cranium (through blood vessels or through burr holes in the skull), the cardiovascular system (heart chambers, coronary arteries, blood vessels), the gastro-intestinal tract (stomach, duodenum, biliary tract, gall bladder, intestine, colon) and the liver, the urinary system (bladder, ureters, kidneys), the pulmonary system (the bronchial tree or blood vessels), the skeletal system (joints), the reproductive tract, and other organs and organ systems.

In one embodiment, the present invention provides real-time computer control to maintain and adjust the position of a treatment system and/or the position of a patient relative to the treatment system. In another alternative embodiment, real-time computer control of the operation of the treatment system itself is provided. Types of treatment systems suitable for use with the present invention include, by way of non-limiting examples, surgical tools and tissue manipulators, devices for in vivo delivery of therapy, such as drugs, angioplasty devices, biopsy and sampling devices, devices for delivery of energy such as RF, thermal or electromagnetic radiation energy, microwave or laser energy or ionizing radiation, and internal illumination and imaging devices, such as catheters, fiber optic transmission and/or receiving systems, endoscopes, laparoscopes, and the like instruments, or a combination thereof.

A general presentation of the present invention will now be described in relation to two preferred embodiments: 1) position tracking for fMRI applications, and 2) position tracking to enable retrospective k-space corrections for reducing motion artifacts in anatomical MRI applications. However, it should be understood by those of ordinary skill in the art that the invention can also be employed with only minor variations that can be provided by one of ordinary skill in the art for other anatomic, physiological and interventional MRI applications.

### Preferred Embodiment One

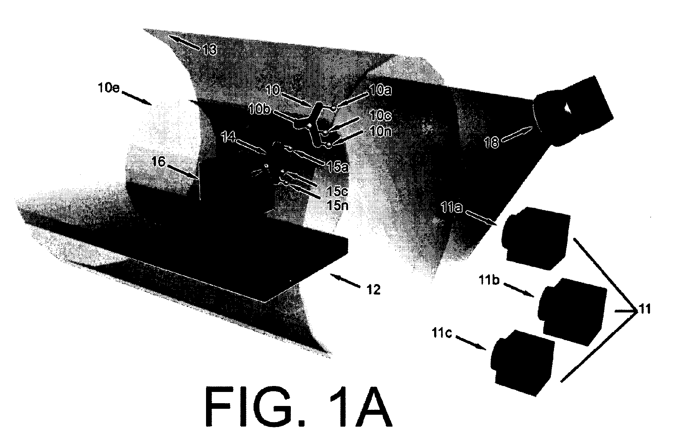

With reference to FIG **1A,** a arrangement. The arrangement is typically not spatially symmetrical, such that the position and orientation of the tool can be uniquely identified in all configurations. The markers, when illuminated, are detectable with high image contrast by the camera system. According to the invention, the reflective markers **10a, 10b, 10c...10n** are not necessarily coplanar rigid reference tool (the tool may be a medically functional or non-functional component of a device) **10** fixed to a stationary target is placed as close as possible to the centre of the measuring volume of an MR-compatible camera system 11, where optimal accuracy and stability are achieved. The reference tool **10** consists of at least three, but potentially more reflective markers **10a, 10b, 10c...10n,** of sufficient size to be identified and resolved in the imaging system. This size can be as small as the resolution of the system allows, by way of non-limiting example, from 1mm to as large as the image field can tolerate without blocking the MRI view. Typically, with present MRI resolution, the size is preferably (but not limited to) approximately 0.5 to 2 cm, especially about 1 cm in size and is provided in a predetermined and preferably precise geometrical and position tracking is improved if the markers are not coplanar. That is, if the markers are each flat circular wafers, the planes of the three wafers are not coincident. They may be coplanar, but preferably two or more are not coplanar with the others, and the respective planes may also be skewed so that the planes are not parallel. The tool **10** is preferably fabricated out of an MRI-compatible material, such as plastic, without ferromagnetic or electrically conductive components, and with magnetic susceptibility close to that of air. Foamed synthetic or composite materials can assist in attaining this property. In the method of the invention, possible locations for the reference tool **10** include the exterior of the transmit/receive head coil **10e** as shown in FIG **1A,** the motorized patient table **12,** or the bore of the magnet **13,** in line-of-sight of the cameras and without obstructing patient positioning.

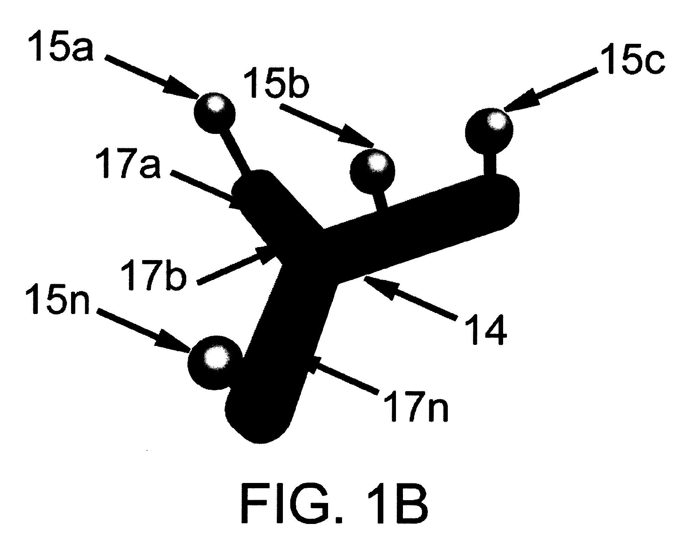

According to one aspect of the invention, a tracking tool **14,** fabricated analogously to the reference tool and comprising an assembly of reflective markers **15a, 15b, 15c...15n** with a different geometry (but which may be independently defined according to variations in geometry allowed and described for the reference markers, such as non-planarity or skewed planes) than the reference tool **10**, so as to allow the camera system software to distinguish between the two tools, is rigidly mounted on a stationary phantom **16**. A "phantom" is, for example, a test object that is filled or built of spatially separated/segregated materials, such as solutions of paramagnetic ions that mimic the MR signal contrast of tissues. The phantom is not instrumental to the medical procedure, but provides a reference point for MR visualization. In a preferred embodiment, the tracking tool (FIG. **1B**) has a minimum of three holes **17a, 17b, ...17n** of precise dimension and location with respect to reflective markers **15a-15n.** The holes are titled (lined, painted, marked, coated, etc.) with an MR contrast agent (e.g., an aqueous or non-aqueous solution, dispersion or suspension of MR contrast agent) to produce strong signal intensity when high spatial resolution MRI (by way of non-limiting example, nominally 1 mm by 1 mm by 1 mm voxel dimension) is performed. The contrast agent should be capable of providing appropriate response to whatever MRI resolution is desired and whatever MRI intensity is used.

With further reference to FIG 1, in one embodiment, a configuration of separated CCD cameras 11, MRI-compatible with respect to ferromagnetic properties and electromagnetic interference at the Larmor frequency of the MRI system, is placed within line-of-sight of the object to be tracked. In accordance with a preferred embodiment of the invention, FIG. 1 illustrates the position and arrangement of said multiple MRI-compatible cameras. At least two cameras **11a** and **11b** are required for tracking with six degrees-of-freedom, although additional cameras **11n** may be used to provide increased accuracy and sensitivity. The cameras may be designed to operate in the visible spectrum, but in the preferred embodiment generally operate in the infrared spectrum so that the tracking system does not affect human vision (e.g., medical personnel who are observing the region either directly or through image-carrying modalities, such as fiber optics or direct view cameras). According to the invention, the source **18** illuminates the two tools with the appropriate and specific light spectrum and can be operated either in pulsed or continuous mode. However, continuous mode is preferable as this is more easily made MR-compatible. Pulsed mode electronics can introduce high-frequency radiofrequency components that interfere with MRI, necessitating filtration and careful selection of pulse frequency.

With further reference to FIG. 1, a high resolution MR image of the phantom **16** with the tracking tool **14** mounted on it is acquired while the camera system **11** tracks the position of both tools **14** and **10.** Further in the method of the invention, the 3D positions of all holes **17a-n** are obtained in relation to the respective spatial coordinates of the MR system and the camera systems. The tracking tool **14** may provide the holes **17a...n** in a two-dimensional array (e.g., the holes lie within a single plane) or a three-dimensional array (eg, at least four of the holes **17a...n** define a pattern wherein at least one hole lies outside of a single plane defined by three other holes. Knowledge of the 3D coordinates of a set of points in two coordinate systems enables the registration transformation between the two coordinate systems to be estimated using a closed-form solution involving quatemions (Horn *J Opt Soc Am A* **4**(4):629-642, 1987). After all necessary information is obtained for the registration of the spatial coordinate frames of the MRI system and camera system, functional MRI experiments can proceed.

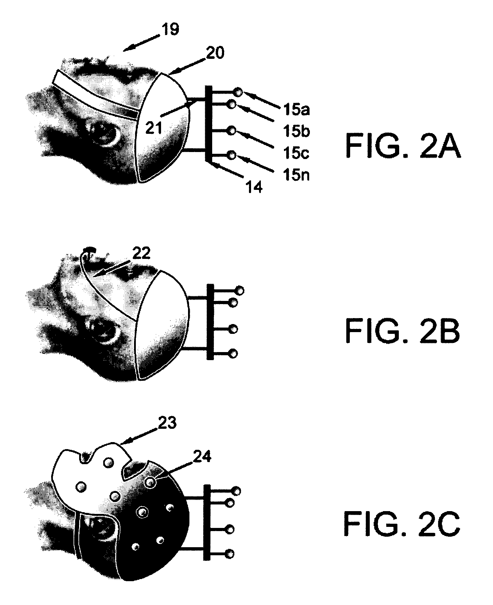

With reference to FIG. **2A,** according to another embodiment of the invention, the second tool with MR contrast markers is mounted on the subject's head **19** using a hat-like device **20.** The mount **20** is shown by way of non-limiting example to contain a spacing element **21** consisting of material (e.g., natural or synthetic polymeric materials, composite materials, etc.) with similar magnetic susceptibility to biological tissues to displace away from the head any magnetic field distortions produced by the tracking tool **14.** In two alternative embodiments, the tool may be placed on the patient's head in a 'bite-mount' **22** or 'molded face-mask' **23** configuration. The bite mount **22**, clenched within the patient's teeth, potentially allows more rigid fixation of the tool with respect to the head. Although requiring patient compliance, this mount is much less aversive than using a bite bar restraint for constraining head motion, as used in some fMRI applications. The molded face mask **23** represents a compromise between the hat-like mount **20** and the bite mount **22**, which attempts to distribute the fixation forces across the head without introducing pressure points and with less compliance required of the patient. Such a mold can be created out of MRI-compatible materials on a patient-specific basis, potentially using available 3D surface scanning technology (e.g., Vivid™ 300, Minolta) coupled with computer-controlled machining equipment. A plurality of holes **24** are incorporated in the mold **23** to prevent the patient from overheating.

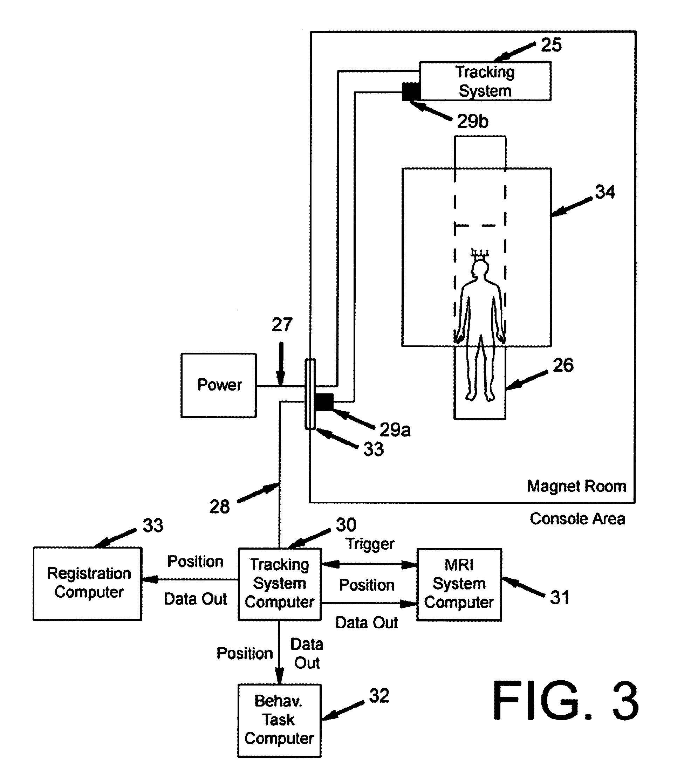

FIG. 3 is a block diagram describing the integration of the camera system with the MRI scanner hardware, according to most aspects of the present invention. In the embodiment shown, the tracking system (cameras plus source) **25** is rigidly mounted at the end of the magnet bore opposite the patient bed **26**. Through shielded cables **27** and **28**, the system **25** is fed power and receives and transmits serial data, respectively. The serial communication cable can also be constructed of optical fiber to reduce the possibility of electromagnetic interference, if two electro-optic conversion modules **29a** and **29b** are included. Direct communication with the tracking system is provided using a tracking system computer **30**, which optionally may be a subsystem of the MRI system computer **31**. The MRI system is configured to send a logic pulse to trigger the onset of position tracking in synchrony with image acquisition, or vice versa. In another embodiment, the tracking system can be operated independently of MRI acquisition to track motions in between scans or during interventional procedures that do not require real-time guidance. The tracking system computer **30** also transmits position data in MRI spatial coordinates to the MRI system computer **31** to allow for retrospective image coregistration, for real-time display of head motion during real-time fMRI, or to adjust imaging gradients such that the imaging scan plane prospectively tracks with moving anatomy (Zaitsev et al., ISMRM Abstr. 2004, 517; Zaitsev et al., ISMRM Abstr. 2004, 2668; Dold et al., ISMRM Abstr. 2004, 742). In a further embodiment, position data can flow to a behavioral task computer **32** to record movement kinematics or motion parameters for use in sensorimotor fMRI experiments. In addition, position data can also flow to an additional registration computer **33** for subsequent alignment of MR images with images from another imaging modality (Elgort et al., ISMRM Abstr. 2004, 957). To optimize position data transmission rate, in one particular embodiment the respective connections between any or all of computers **30, 31, 32,** and **33** are high speed internet connections rather than serial connections.

The present invention thereby provides improved registration of MRI images within the same examination session. For example, in an fMRI examination, registration of 3D anatomical neuroimages with fMRI time series image data is enabled. However, the method of the invention also permits the imaging scan plane and field-of view offset to be adjusted such that the start of each time series run or the acquisition of 3D anatomical data are initially registered spatially.

For anatomical imaging applications, the use of reference and tracking tools according to the present invention enables position measurements to be obtained in image spatial coordinates during the time period for image acquisition for the purpose of intra-image correction. In the method of the invention, such corrections entail assigning the appropriate slice location for phase encoding data in multislice imaging, or assigning a displacement-dependent phase shift or rotation to k-space data acquired in a 3D acquisition. The latter embodiment would also typically require use of an additional gridding algorithm to account for non-rectilinear sampling of k-space. According to the invention, upon gridding, Fourier transformation of the motion-corrected k-space data would yield an improved image (see Preferred Embodiment Two). An alternative approach of the present invention involves providing the patient with visual feedback of their head motion, using an MRI-compatible visual display system, and instructing said patient to attempt to keep all six degrees-of-freedom motion to a minimum during anatomical scanning. In the method of the invention, visual feedback can also be used to instruct subjects to align their head in coregistration with previously acquired MR image data

In another embodiment, the tracking system **25** can be positioned near the patient entrance to the magnet. In this configuration, position tracking of other moving anatomy is possible, necessitating that the tracking tool is fixed to the appropriate skin surface using conforming apparel. Examples where 2D or 3D motion correction can be adopted include measurement of respiratory motion on the abdomen or other regions of the trunk, including the shoulder, to improve image quality in musculoskeletal MRI, or to record movement from swallowing. Such measurements also permit use of conventional gating strategies for respiratory motion, or the rejection of motion contaminated data with repeated scanning until all desired spatial frequencies of the tissue of interest are encoded. In gating applications, only relative position measurements are required so that the initial calibration procedure can be avoided.

Regardless of the location of the tracking system, the present invention provides for direct measurement of anatomy and objects of interest during MRI. In one embodiment, the tracking tool must be appropriately fixed to the skin surface or the surface of a tool or probe. Examples include measuring the motion of the patient table to improve scan plane prescription and localization during high resolution images, measuring wrist position for a patient wearing an MRI-compatible data glove for fMRI examinations of motor function of the hand, stereotactic placement of an interstitial probe in an interventional MRI application, or placement of external ultrasound applicator on the skin surface to improve localization of the focal zone of heating in thermal therapy of tumors.

In another embodiment of the invention, MR images from different examination sessions can be co-registered. According to the invention, the calibration procedure involving the reference tool provides a method of recording the absolute position of the tracking tool as a function of time. Since the tracking tool is visible in MR images, it is possible to combine the tracking system with image-based coregistration approaches to co-register data from different examination sessions in absolute coordinates. This embodiment, which accounts for the possibility that the tracking tool may be located in a slightly different spatial location on different examination sessions, has a variety of useful applications. For example, the relative difference in head positions can be calculated for coregistration purposes. Alternatively, within examinations, the scan plane and field-of-view offset can be adjusted to ensure that images are acquired with intrinsic registration at the start of image acquisition. According to the invention, it is also possible to use the position tracking system in combination with MRI-compatible stepper motors to adjust the orientation of the head within a head coil. In yet another embodiment, the subject may be provided with visual feedback of their six degree-of-freedom head motion using an MRI-compatible display, and instructed to orient their head to align with MRI data acquired on a previous examination. It is recognized that this may be a difficult task for subjects with neurological impairment. Nevertheless, for particularly taxing applications where subtle changes in neuroanatomy are to be detected across examination sessions, this application of the invention ensures that imaging of the head is conducted in exactly the same position within the MRI system. Thus, subtle sources of variability such as partial voluming of neuroanatomy within imaging voxels, placement of the head within the non-uniform sensitivity profile of the head coil, and differences in susceptibility artifact due to different head position in the MRI system are all eliminated by the method of the present invention.

The method of the invention will now be further described by way of a detailed example with particular reference to certain non-limiting embodiments and to the accompanying drawings in FIGS. **1** to **8**. This work has previously been presented in preliminary form (Tremblay et al., ISMRM Abstr. 2003; 385).

Experiments were conducted using a whole-body MRI scanner (Signa, General Electric Medical Systems, Waukesha, WI; LX 8.5 software platform; CV/i hardware platform) with a standard quadrature birdcage headcoil, an infrared position tracking system housing two CCD cameras illuminated by infrared-emitting diodes (Polaris, Northern Digital, Inc.; enhanced electromagnetic interference option), and two precision-machined plastic tools with infrared-reflective markers (Traxtal, Inc., Toronto, Ontario). The tracking system includes infrared-emitting diodes to provide illumination. The tracking system was positioned within the magnet room as shown in FIG. **3**. This configuration ensured that the two tools remained continuously within the measuring volume of optimal accuracy of the tracking system, situated approximately 1.5 m away from the face of the cameras. Measurements within the MRI system indicated high accuracy with a precision of less than 100 microns in displacement, and nominally 0.1 degrees in rotation. The tracking system was made MRI-compatible by relocating the DC-to-DC converter stage (power supply) from inside the Polaris unit to outside of the MR room. Improved shielding of all parts of the tracking system, including the cables, was added using aluminum foil to ensure effective suppression of electromagnetic interference with the MR imaging process. The tracking system's cables entered the MR room through the filtered penetration panel. Control was provided using a laptop computer over a standard serial port interface to adjust settings and to receive tracking data.

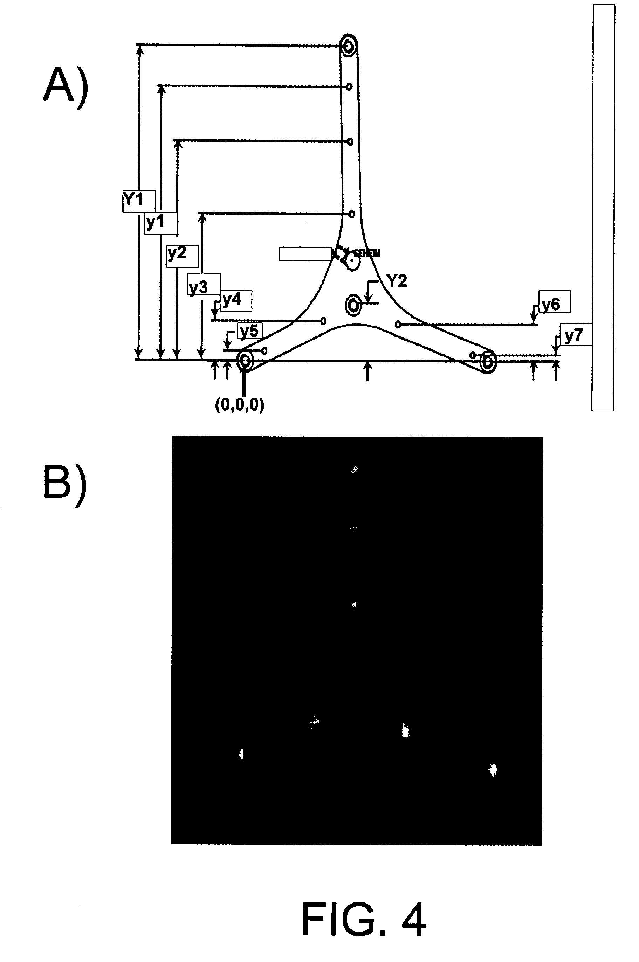

Before initiating the fMRI study, a calibration procedure was performed to convert head motion information (initially in tracking system coordinates) into the spatial coordinates of the MR system An example of a single high resolution MR ''top view" image taken from a 3D acquisition of the tracking tool with its holes filled with a solution of Gd-DTPA contrast agent (Magnevist, Burlex - 1:100 dilution by volume) is shown in FIG. **4B,** together with a diagram of the tool geometry (FIG. **4A**). FIG. **4A** also indicates the origin of the spatial coordinates for the tool, and representative spatial coordinates of the holes (yᵢ) and markers (Yᵢ). The MR image localizes the 3D positions of the centers of the different holes (7 holes in this case) obtained in the spatial coordinates of the MR system. Image quality was sufficient to achieve accurate calibration (see below).

The following scan parameters were used for MRI:
High resolution MR anatomical acquisition
   - Slice Thickness: 0.7mm
   - Matrix: 512 X 512
   - 60 slices
   - TE/TR/θ = 7ms/35ms/35 deg
   - Field of view : 22cm X 22cm
Conventional MR anatomical acquisition
   - Slice Thickness = 1.4mm
   - Matrix 256 X 128
   - 124 slices
   - TE/TR/θ = 6ms/35 ms/35 deg
   - Field of view : 22cm X 22cm
Spiral fMRI acquisition
Slice Thickness = 5mm
   - Matrix 64 X 64
   - 20 slices
   - TE/TR/θ = 40ms/2sec/80 deg
   - Field of view: 20cm X 20cm
Block design
   - 20 sec task (bilateral alternating finger tapping + tracking (path-length=2mm, cursor velocity=0.1 mm/sec))
   - 2 sec cue
   - 20 sec rest
   - 2 sec cue

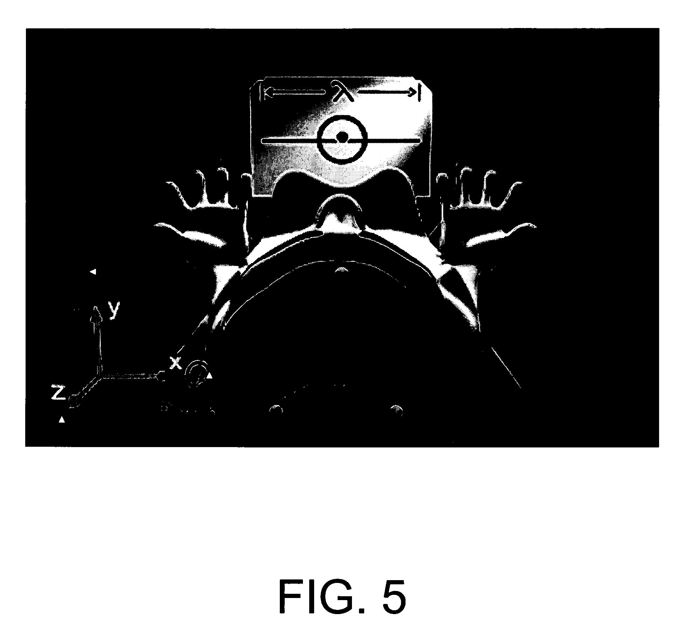

The utility of the invention was then tested in an fMRI experiment, using an elastic cap to fix the tracking tool to the head. A young healthy adult subject consented to participate in a block-design fMRI experiment, consisting of alternating 20-second blocks of rest (where the subject was instructed to remain still and to perform fovial fixation at a centrally-located crosshair) and a task of equal time duration. The display was visualized using an LCD-projector mounted outside the magnet room in the console area such that it back-projected images onto a screen mounted at the opening of the magnet bore. The subject viewed this display using the angled mirrors within the head coil. The task consisted of bilateral alternating finger tapping while tracking a moving target on the display. A view from the perspective of the tracking system is shown in FIG. 5, with the head coil and reference tool omitted for visual clarity. A hollow circle moved back and forth (corresponding to the subject's left-right direction) on the horizontal line of the projected display during the task blocks. The extent of the horizontal line, denoted λ, was nominally equivalent to 10 degrees visual angle, scaled to 1 mm head motion (peak-to-peak) in the left-right direction. During each 20-second block, the hollow circle tracked the full extent of the horizontal line in one cycle at a constant velocity corresponding to left-right head motion of 0.1 mm/s. A black filled circle represented the left-right head motion produced by the subject. The subject was instructed to try to keep the filled circle within the hollow circle during the task blocks, while performing self-paced finger tapping.

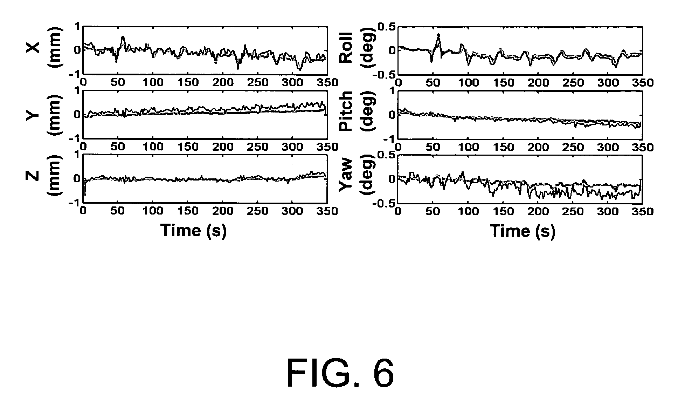

Shown in FIG. 6 are plots of the head motion in six degrees-of-freedom translation along the three orthogonal directions (x, y, z), and rotation about the three orthogonal directions (roll, pitch, yaw), obtained with the tracking system (converted into coordinates of the MR system). For comparison purposes, analogous six degrees-of-freedom motion estimates are also shown for the case where an image-based coregistration algorithm available in Analysis of Functional Neuroimages (AFNI), an established freeware package designed for fMRI data processing (Cox, Neurolmage. *Comp Med Res* 1996, **29**:162-173), is used to align the time series data spatially. The camera-based (black) and image-based (gray) motion estimates are in close agreement, with maximum differences in displacement that are well below 1 mm, and maximum differences in rotations that are well below 0.5 degrees.

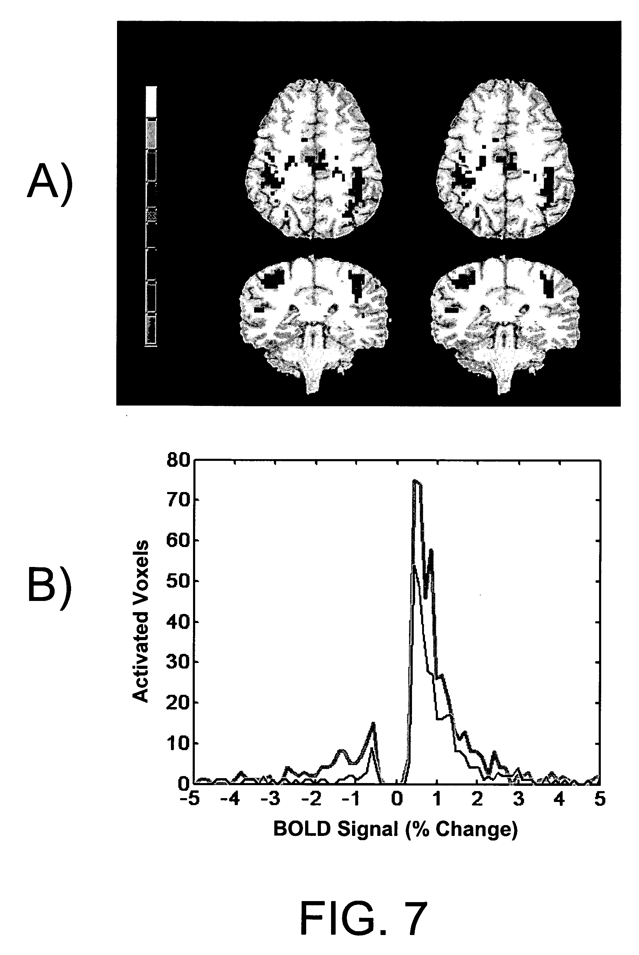

FIG. 7A shows representative images of brain activity (axial and coronal views) associated with this experiment. The two maps to the left correspond to those obtained after registration with the image-based coregistration algorithm in AFNI, and the two to the right to those obtained after coregistration with the tracking data obtained using the camera-based system. The pattern of brain activity observed was as expected. The task performed by the subject engaged a network of brain regions typically involved in sensorimotor tracking, including the primary somatosensory and motor cortex bilaterally, as well as the supplementary motor area and the premotor and parietal cortical regions. The only difference between the maps, in terms of the processing steps, is the coregistration approach. The maps were obtained in AFNI using the following steps after coregistration:
1) 3-point median temporal filtering
2) Gaussian spatial blurring (full-width-at-half maximum=4mm)
3) Time-series detrending to remove baseline offsets and linear trends over the duration of the experiment
4) Masking, such that signal intensity outside of the brain equaled zero
5) Boxcar cross correlation functional analysis (correlation coefficient CC_{TH} = 0.39; p = 3.4 × 10⁻⁷; which includes Bonferroni correction for multiple statistical comparisons).

The results of this test demonstrate that the images of brain activity obtained using the camera-based tracking system are very consistent with images obtained using the image-based coregistration algorithm, indicating that the tracking system works well for the purposes of retrospective coregistration for human fMRI studies. Further analysis of the activation images also shows that image-based coregistration and camera-based coregistration perform equally well for fMRI tasks. Both data sets show essentially the same histograms of activated voxels versus fMRI BOLD signal expressed as mean percentage signal change (FIG. **7B**) although there is slightly less activation obtained with the camera-based system (black) compared to that obtained by image-based coregistration (gray). The small differences between the two approaches could either be due to noise in the tracking data (FIG. **6**), or sensitivity of the image-based coregistration to factors such as image signal-to-noise ratio, or eye motion.

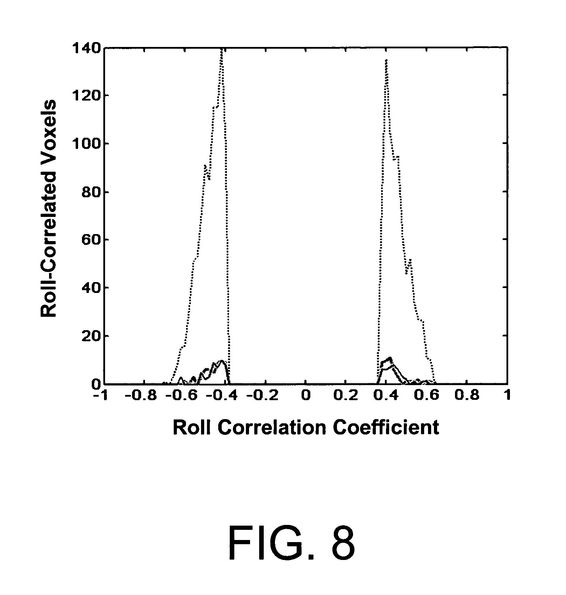

An additional evaluation involved voxel-wise correlation of the time-series of fMRI signal intensity values with the roll motion information (dominant rotation during the tracking task) obtained using the camera-based system. This allowed estimation of the amount of task-correlated motion still present following coregistration. The number of brain voxels significantly correlated with roll motion is reported in FIG. **8,** using the same histogram approach and correlation threshold CC_{TH} = 0.39 as for the functional maps in FIG. **7.** Only voxels with correlation values that exceed this statistical threshold are considered. Both image-based and camera-based coregistration greatly decrease the amount of task-correlated motion, as shown in FIG. **8,** approximately ten-fold in comparison with the case where coregistration is not performed. Again, both coregistration approaches appear to work equally well.

### Preferred Embodiment Two

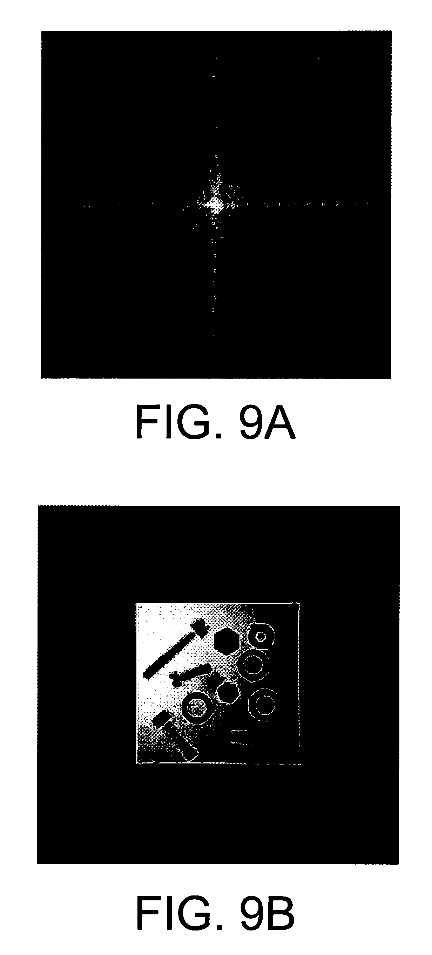

The method of the invention will be further described by way of a detailed example with particular reference to certain non-limiting embodiments and to the accompanying drawings in FIGS. 9 to 12. This embodiment used the same MRI scanner configuration and tracking camera system outlined in detail in the experiments described in Preferred Embodiment One.

In this experiment, the effectiveness of the camera-based tracking system was demonstrated for retrospective correction of motion artifact in k-space as applied to anatomical MR imaging. Conventional rectilinear k-space readouts, assuming a static object, collect samples on an evenly spaced Cartesian grid. For a moving object, according to the shift and projection theorems of the Fourier Transform, distortions are introduced in k-space by the incorrect assumption that the data lie on the same Cartesian grid. Subsequent motion artifacts are introduced in MR images on Fourier Transformation. To demonstrate correction of this problem, 2D imaging was performed on a rotating phantom while motion was tracked using the camera system. The motion data were then used to correct for the actual k-space trajectory prior to image reconstruction.

An acrylic phantom (4 x 4 x 4 inches in size) was constructed and filled with an agar gel doped with Gd-DTPA contrast agent (Magnevist, Burlex). The gel was created using 1 mL Magnevist, 500 mL of distilled water, and 5 g of agar powder. A selection of plastic nuts, bolts, and washers was inserted into the gel to provide edge details and image contrast. The phantom was rotated by an ultrasonic MR-compatible stepper motor (MTL Microtech Laboratory Inc. Japan) mounted at the end of the magnet bed. The stepper motor was controlled from a console hosting LabVIEW 6 (National Instruments, Austin, TX), and was coupled to the phantom by a customized shaft linkage system. The shafts were attached to a rod extending from the centre of either end of the phantom, free to rotate over a rotisserie-like support. The phantom was positioned centrally in the standard quadrature transmit/receive birdcage head-coil. The motion tracking system was set up in the same fashion as in Preferred Embodiment One, with the tracking tool attached to a rod centered on the backside of the phantom. In the present experiment, roll rotation was imparted to the phantom back and forth about the longitudinal axis of the magnet by 17.34 degrees, beginning in the clockwise direction, for one cycle during k-space data acquisition.

The following scan parameters were used for MRI:
Calibration of tracking system: 2D T1-weighted Fast SPGR
   - Slice thickness: 4.0 cm
   - Matrix: 256 X 160
   - Slices: 20
   - TE/TR/theta = 5.4 ms/400 ms/35 deg
   - Field of view: 24 cm
Anatomical acquisition: 2D T1-weighted SPGR
   - Slice thickness: 4.0 cm
   - Matrix: 256 X 256
   - Slices: 1
   - TE/TR/theta = 6.9 ms/400 ms/35 deg
   - Field of view: 20 cm

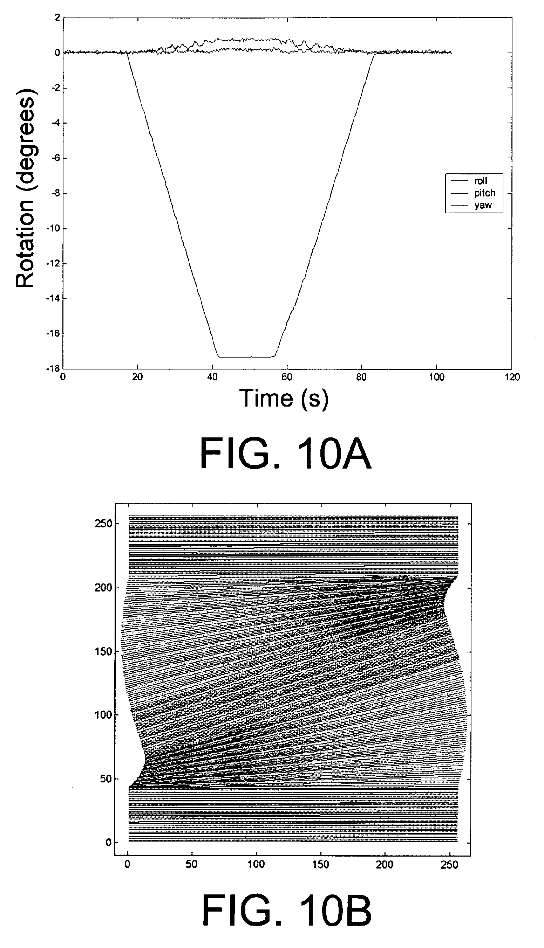

The k-space data (log 10 of magnitude) and the reconstructed reference image of the static phantom are shown in FIG. **9A** and **9B**. Motion data were subsequently collected at a sampling rate of 4.5 Hz, were transformed to the spatial coordinates of the MRI system, and interpolated to determine the position of the phantom for each time point associated with the 256 x 256 k-space samples (FIG. **10A**)**.** Some pitch and yaw rotations were observed, indicating that the phantom did not rotate purely with 1 degree of freedom, but these other rotations were small and not included in the subsequent correction scheme. Applying the rotation estimates to the ideal rectilinear k-space trajectories provided the set of corrected k-space trajectories (FIG. **10B**)**.** Based on the Fourier projection theorem, each trajectory point was rotated about the centre of k-space by the same amount of rotation accrued by the phantom at that specific time.

The k-space data, along with the corrected trajectories, were then passed through a reconstruction server for 2D gridding (Jackson et al. IEEE Transactions on Medical Imaging 1991; **10**:473-478; 1991). This procedure is necessary to.enable reconstruction of MR images from non-Cartesian k-space trajectories using the computationally efficient Fast Fourier Transform Briefly, a kernel is convolved with the complex k-space data associated with the points along each corrected trajectory, and the result is resampled onto a Cartesian grid. Inhomogeneous sampling densities in k-space were then corrected by gridding a unity matrix with the corrupted trajectories. This latter procedure created a density map wherein high density regions were assigned a weighting factor greater than 1, and low density regions were assigned a weighting factor less than 1. Dividing the corrected k-space information by the computed density map produced the final k-space data.

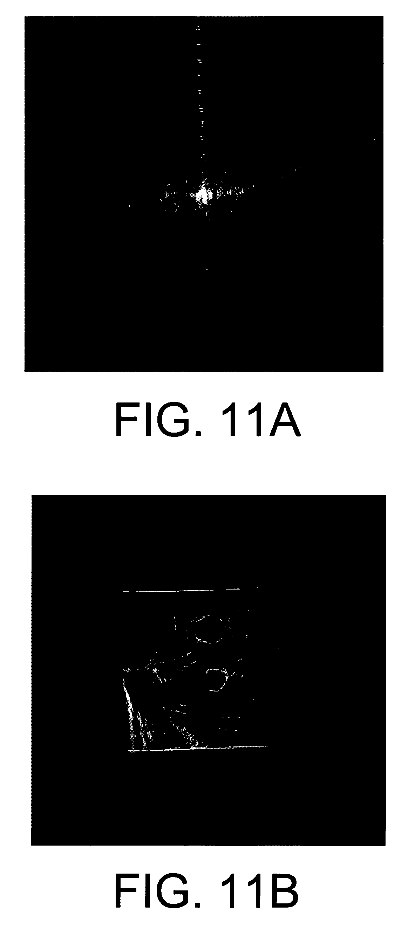

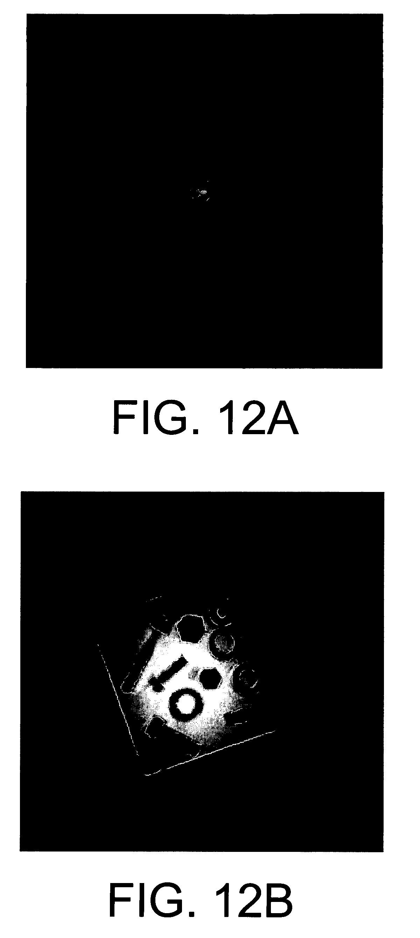

FIGURES **11A** and **11B** show the uncorrected k-space data and reconstructed MR image obtained with the phantom rotating during data acquisition. Motion artifacts are clearly observed. FIGURE **12A** shows the corrected k-space data computed based on the tracking data shown in FIG. **10A.** Although rotated, the results strongly resemble those for the static phantom shown in FIG. **9A.** Importantly, there are some missing portions of k-space due to the specific nature of the motion applied. Interpolation strategies can be developed to reduce this problem.. The associated motion-corrected image (FIG. **12B**) shows negligible motion artifact on visual inspection. Some minor spatial blurring is observable, an inevitable component of the gridding procedure. Nevertheless, the corrected MR image is an excellent representation of the reference (FIG. **9B**). This retrospective procedure may be applied to any set of k-space trajectories in the case of rigid-body motion, and sets the stage for developing an analogous 3D motion correction algorithm.

The empirical data summarized above demonstrate that the camera-based position tracking system disclosed in the present invention can be applied in a very flexible way to assist in the use of motion measurements to improve the quality of fMRI and MRI data. Additional experiments can be designed and implemented that support the feasibility of the various applications and embodiments associated with the invention as disclosed herein.

The patent and literature references discussed above and the following U.S. Patents and documents are incorporated herein by reference for their teaching of background technology relating to the field of the present invention: 4,716,368, Haacke; 4,761,613, Hinks; 5,111,820, Axel et al.; 5,271,400, Dumoulin et al.; 5,323,110, Fielden et al.; 5,570,019, Moonen et al.; 5,771,096, Anderson; 5,307,808, Dumoulin et al.; 5,425,367,Shapiro et al.; 5,558,091, Acker et al.; 5,913,820, Bladen et al.; 6,016,439, Acker; 5,899,858, Muthupillai et al.; 5,545,993, Taguchi et al.; 5,797,396, Geiser et al.; 5,953,439, Ishihara et al.; 6,067,465, Foo et al.; 6,157,677, Martens et al.; 6,292,683, Gupta et al.; 6,317,616, Glossop ; 6,725,080, Melkent et al.
1. E Seto, G Sela, WE McIlroy, SE Black, WR Staines, MJ Bronskill, AR McIntosh, and SJ Graham. Quantifying Head Motion Associated with Motor Tasks used in FMRI. *Neurolmage* **14**:284-297 (2001)
2. JV Hajnal, R Myers, A Oatridge, JE Schwieso, I.R. Young, G.M. Bydder. Artifacts due to stimulus correlated motion in functional imaging of the brain. *Magn Res Med* **31**: 283-291 (1994)
3.KJ Friston, SCR Williams, R Howard, RSJ Frackowiak, R Turner. Movement related effects in fMRI time series. *Magn Res Med* **35**:346-355 (1996)
4.Dumoulin CL et al. Real-time position monitoring of invasive devices. Mag Reson Med 1993; **29:** 411-415.
5. ET Bullmore, MJ Brammer, S Rabe-Hesketh, VA Curtis, RG Morris, SCR Williams, T Sharma, and PK McGuire. Methods for Diagnosis and Treatment of Stimulus-Correlated Motion in generic Brain Activation Studies using FMRI. *Human Brain Mapping* **7**: 38-48 (1999)
6. P.A. Bandettini, A Jesmanowicz, EC Wong, JS Hyde. Processing Strategies for Time-Course Data Sets in Functional MRI of the Human Brain. *Magn Res Med* **30:** 161-173 (1993)
7. RW Cox. AFNI: Software for Analysis and Visualization of Functional Magnetic Resonance Neurolmage. *Comp Med Res* **29**:162-173 (1996)
8. RW Cox, A Jesmanowicz. Real-Time 3D Image Registration for Functional MRI. *Magn Res Med* **42:**1014-1018 (1999)
9. S Grootoonk, C Hutton, J Ashburner, AM Howseman, O Jospehs, G Rees, KJ Friston, and R Turner. Characterization and Correction of Interpolation Effects in the Realignment of FMRI Time-Series. *Neurolmage* **11**:49-57 (2000)
10. A Babak et al. A quantitative comparison of motion detection algorithms in fMRI. *Magn Res Im* **19**:959-963 (2001)
11. K Voklye et al. Motion Correction in fMRI via Registration of Individual Slices into an Anatomical Volume. *Magn Res Med* **41**:964-972 (1999)
12. BKP Horn. Closed Solution of Absolute Orientation using Unit Quaternions. *J Opt Soc Am A* **4**(4):629-642 (1987)
13. Coutts GA et al., Integrated and Interactive Position Tracking and Imaging of Interventional Tools and Internal Devices Using Small Fiducial Receiver Coils. Magnetic Resonance in Medicine **40**:908-13 (1998)
14. Daniel BL et al. Comparison of optical and MR-tracking methods for scan plane guidance during dynamic MR imaging of the spine ISMRM Abstr. 1997; 1928.
15. Bomert P et al. In-plane position tracking of medical instruments during MRI. ISMRMAbstr. 1997 ; 1925.
16. M. Zaitsev et al. Imaging of Freely Moving Objects by Means of Real-Time Image Coordinates Update Using an External Optical Motion Tracking System. ISMRM Abstr. 2004;2668.
17. M. Zaitsev et al. Prospective Real-Time Slice-by-Slice 3D Motion Correction for EPI Using an External Optical Motion Tracking System. ISMRM Abstr. 2004; 517.
18. C. Dold et al. Updating of MRl Gradients Using a Infrared Tracking System to Compensate Motion Artifacts. ISMRM Abstr. 2004; 742.
19. D.R Elgort et al. MR Respiratory Motion Tracking for Use With An Augmented Reality Surgical System. ISMRM Abstr. 2004; 957.
20. M. Tremblay and S.J. Graham. A Comparative Study of fMRI Head Motion obtained with a Retrospective Coregistration Algorithm versus External Monitoring. ISMRM Abstr. 2003: 385.
21. J.I. Jackson et al. Selection of a Convolution Function for Fourier Inversion Using Gridding. IEEE Transactions on Medical Imaging **10**:473-478 (1991).

It should be understood that the foregoing description is merely illustrative of the invention. Various alternatives and modifications can be devised by those skilled in the art without departing from the scope or spirit of the invention. Different equipment, methodologies, software, algorithms and the like may be selected by the ordinarily skilled artisan to perform the methods and construct apparatus within the scope of the present invention. Accordingly, the present invention is intended to embrace all such alternatives, modifications and variances which fall within the scope of the appended claims.

## Claims

1. An optical image-based motion tracking method for determining the location and orientation of at least one object moving through three-dimensional space within or on the surface of a human or non-human body undergoing magnetic resonance (MR) imaging, the method comprising
(a) obtaining 3D coordinates of the at least one object within a field-of-view of said MR imaging system using a plurality of MR-compatible cameras;
(b) obtaining motion information coordinates with the optical tracking system;
(c) converting motion information coordinates obtained with the optical tracking system into coordinates of said MR imaging system;
(d) acquiring a motion information file for each MR imaging scan of the body;
(e) converting said motion information file into coordinates of the MR imaging system using a registration transformation;
(f) applying each converted motion information file to realign its corresponding MR time series of images; and
applying each converted motion information file and corresponding MR time series of images to track movement of the at least one object in the field-of view.

2. The method of claim 1, wherein the at least one object comprises biological materials in a human or non-human body.

3. The method of claim 1, wherein the at least one object comprises at least one medical device used in diagnostic and interventional medical and surgical procedures.

4. The method of claim 3 wherein the at least one medical device is selected from the group consisting of surgical tools and tissue manipulators, devices for in vivo delivery of drugs, angioplasty devices, biopsy and sampling devices, devices for delivery of energy or radiation, and internal illumination, imaging devices, tools, instruments, devices, and chemical agents used in conventional anatomic MR imaging or functional MRI studies.

5. The method of claim 1, wherein the camera system estimates motion with six degrees of freedom of a rigid tool containing multiple precisely located reflective objects, the camera system comprises an MR-compatible current charge- coupled-device camera.

6. The method of claim 5, wherein the camera system provides an image with high spatial resolution and wherein the camera system operates at video frame rates and/or the camera system provides measurement accuracy and precision below 100 microns.

7. The method of claim 6, wherein the camera system uses a low-intensity pulsed or continuous light source.

8. The method of claim 7, wherein the camera system is sensitive to at least one specific optical wavelengths outside the range of human vision.

9. The method of claim 1, wherein the optical tracking system transmits position data in MRI spatial coordinates to an MRI system computer to allow for retrospective image coregistration and optionally The optical tracking system transmits the position data in the MR imaging spatial coordinates for real-time display of head motion during real-time fMRI.

10. The method of claim 9, wherein the optical tracking system transmits the position data in the MR imaging spatial coordinates to adjust imaging gradients such that the imaging scan plane prospectively tracks with moving anatomy or the optical tracking system collects data from multiple different imaging modalities to be registered by measuring the patient's orientation in each image with respect to a common coordinate system.

11. The method of claim 10, wherein the data from the optical tracking system flows to a behavioral task computer to record movement kinematics or motion parameters for use in sensorimotor fMRI performances.

12. The method of claim 10, wherein the position data flows to an additional registration computer for subsequent alignment of MR images with images from another imaging modality.

13. The method of claim 10, wherein the transmission of said position data between any or all said computers are made by high speed internet connections .

14. The method of claim 13, wherein the imaging scan plane and field-of-view offset is adjusted such that the start the acquisition of 3D anatomical data are initially registered spatially with respect to previous image acquisition.

15. The method of claim 14, wherein the real-time computer control tracks the position of the interventional treatment system, including at least one element selected from the group consisting of surgical tools and tissue manipulators, devices for in vivo delivery of drugs, angioplasty devices, biopsy and sampling devices, devices for delivery of energy or radiation, internal illumination devices and imaging devices.

16. The method of claim 15, wherein the tracking system is operated independently of MRI acquisition to track motions in between scans or during interventional procedures that do not require real-time guidance.

17. The method of claim 1, wherein the optical position tracking system is used in an interventional MRI application where images are used to guide and monitor minimally-invasive diagnostic and therapeutic procedures or the optical position tracking system is used in a medical application to provide registration of MRI data and with data obtained from imaging modalities other then MRI.

18. The method of claim 1, wherein the optical position tracking system is used to longitudinally evaluate changes in brain images acquired over time periods selected from the group consisting of minutes, hours, days, weeks, and months.

19. The method of claim 1, wherein the optical position tracking system is independent of human operator input.

20. The method of claim 1, wherein the optical position tracking system is not reliant on pixel size.

21. The method of claim 1, wherein the position tracking system is insensitive to signal variations in and between MR images with respect to position measurement.

22. The method of claim 1, wherein operation of the position tracking device is independent of the MR scanner.

23. The method of claim 1, wherein the motion tracking system is used to determine the position of anatomy as a function of a scanning session to enable coregistration of image data and to detect and quantify changes caused by motion.

24. The method of claim 1, wherein the motion tracking system assists positioning of the body so relative to MRI system so that MRI scans are performed with the anatomy in the same location within the MRI scanner on each session or wherein the motion tracking system assists in providing data for positioning the body during MR imaging to provide the same spatial resolution and orientation as between different examinations.

25. The method of claim 1, wherein the optical position tracking method is used to co-register neuroanatomical MRI with fMRI images of brain activity.

26. The method of claim 1, wherein the optical position tracking system tracks position and the position tracking is used to validate image-based coregistration algorithms.

27. The method of claim 15, wherein the optical position tracking system operates with real-time computer control to sense and maintain the position of an interventional treatment system for use with objects selected from the group consisting of surgical tools and tissue manipulators, devices for in vivo delivery of drugs, angioplasty devices, biopsy and sampling devices, devices for delivery of energy or radiation, internal illumination devices and internal imaging devices.

28. The method of claim 1, wherein the optical tracking system uses a local pattern matching technique dependent on known geometry of tools within the MRI field that contain optically reflective markers, the optical tracking system being insensitive to signal variations in and between MR images and local pattern matching technique is used when MR signal variations are related to flow effects, motion effects, or wash-through of contrast agents.

29. The method of claim 22, wherein a zone of optimal accuracy and sensitivity of said position tracking device is made independent of the MRI field-of-view and wherein the zone of optimal accuracy and sensitivity of the position tracking device is made larger than a 45 cm field-of-view.

30. The method of claim 17, wherein position measurements made with the optical position tracking device are used to track motions in a fringe magnetic field of the scanner.

31. The method of claim 15, wherein the independent position-tracking device is used to validate other approaches for motion measurement and correction on the basis of simulated data sets or by comparison with other more established algorithms.

32. The method of claim 1, wherein the position tracking system provides intra-acquisition motion information and wherein the intra-acquisition motion information includes measurement of positional displacement that occurs during the acquisition of a certain image.

33. The method of claim 21, wherein the accuracy of said position monitoring system has properties elected from the group consisting of a) independence of MR image quality, b) being unaffected by inhomogeneity of a main field of view, c) being unaffected by nonlinearity of the MR gradients, and being unaffected by tissue nonzero magnetic susceptibility

34. The method of claim 9, wherein the position tracking system is used to provide visual feedback of head position and orientation during anatomical MR to help prevent head motion.

35. An optical image-based motion tracking method for determining the location and orientation of at least one object moving through three-dimensional space within or on the surface of a human or non-human body undergoing magnetic resonance (MR) imaging, comprising:
(a) obtaining 3D coordinates of the at least one object within a field-of-view of the MR imaging system using a plurality of MR-compatible cameras;
(b) obtaining motion information coordinates with an optical tracking system;
(c) converting the motion information coordinates obtained with the optical tracking system into coordinates of said MR imaging system;
(d) acquiring a motion information file for each MR imaging scan;
(e) converting the motion information file into coordinates of the MR imaging system using a registration transformation;
(f) applying each converted motion information file to realign its corresponding functional MRI time series of images; and
(g) applying each converted motion information file and corresponding functional MR time series of images to accurately track movement of the at least one object in said Field-of-view.

36. An optical image-based motion tracking method for determining the location and orientation of at least one object moving through three-dimensional space within or on the surface of a human or non-human body undergoing magnetic resonance (MR) imaging comprising:
(a) obtaining 3D coordinates of the at least one object within a field-of-view of the MR imaging system using a plurality of MR-compatible cameras;
(b) obtaining motion information coordinates with an optical tracking system;
(c) converting the motion information coordinates obtained with the optical tracking system into coordinates of the MR imaging system;
(d) acquiring a motion information file for each MR imaging scan;
(e) converting the motion information file into coordinates of the MR imaging stem using a registration transformation;
(f) applying each converted motion information file to correct or augment a corresponding MR anatomical time series of images;
(g) applying each converted motion information file and corresponding MR anatomical time series of images to track movement of the at least one object in said field-of-view.

37. An optical image-based motion tracking method for determining the location and orientation of at least one object moving through three-dimensional space within or on the surface of a human or non-human body undergoing magnetic resonance (MR) imaging, comprising:
(a) obtaining 3D coordinates of the at least one object within a field-of-view of the MR imaging system using a plurality of MR-compatlble cameras;
(b) obtaining motion information coordinates with an optical tracking system;
(c) converting the motion information coordinates obtained with the optical tracking system into coordinates of the MR imaging system;
(d) acquiring a motion information file for each MR imaging scan;
(e) converting the motion information file for each MR imaging scan into coordinates of the MR imaging system using a registration transformation;
(f) applying each converted motion information file to correct or augment a corresponding interventional MRI time series of images; and
(g) applying each converted motion information file and corresponding interventional MRI time series of images to accurately track movement of the at least one object in the field-of-view.

38. A multi-modality imaging system comprising a motion tracking system, an MRI system and a tool that is responsive to MRI signals that can be tracked in three dimensions in coordinates of the MRI system, the motion tracking system being referencable in time against images taken by MRI so that motion effects in an MRI image can be corrected.

39. The imaging system of claim 38 wherein the tool comprises a device having holes that are marked with an MRI responsive material.

40. The system of claim 39 wherein movement of the tool is tracked to provide information on the movement of a body segment imaged by the MRI containing or supporting the tool without any free range of movement independent of the body segment.

41. An optical image-based motion tracking method for determining the location and orientation of at least one object moving through three-dimensional space within or on the surface of a human or non-human body undergoing magnetic resonance (MR) imaging, the method comprising:
(a) obtaining 3D coordinates of the at least one object within a field-of view of said MR imaging system using a plurality of MR-compatible cameras;
(b) obtaining motion information coordinates with the optical tracking system;
(c) converting motion information coordinates obtained with the optical tracking system into coordinates of said MR imaging system;
(d) acquiring a motion information file for each MR imaging scan of the body;
(e) converting said motion information file into coordinates of the MR imaging system using a registration transformation;
(f) applying each converted motion information file to realign its corresponding MR time series of images; and
applying each converted motion information file and corresponding MR time series of images to effect position tracking to enable retrospective k-space corrections for reducing motion artifacts in anatomical MRI applications.
